# EUROPEAN PATENT APPLICATION

(11) **EP 4 748 309 A1**
(43) Date of publication of application: **27.05.2026**
(21) Application number: 25217380.2
(22) Date of filing: 20.11.2025
(51) Int. Cl.: A61B 5/145

(54) **MANUFACTURING APPARATUS FOR SENSOR DEVICE AND MANUFACTURING METHOD FOR SENSOR DEVICE**

(30) Priority: 20.11.2024 KR 20240165846
(71) Applicant: i-Sens, Inc., Seoul 06646 (KR)
(72) Inventor: HA, Jung Uk, 06646 Seoul (KR)
(74) Representative: Isarpatent

(57) **Abstract**

According to one aspect of the present invention, a manufacturing apparatus for a sensor device and a manufacturing method for a sensor device may be provided, which may effectively prevent the occurrence of manufacturing defects during the manufacturing of the sensor device, and may effectively improve workability, productivity, and economic feasibility during the manufacturing of the sensor device.

## Description

### BACKGROUND OF THE INVENTION

### Field of the Invention

The present invention relates to a manufacturing apparatus for a sensor device and a manufacturing method for a sensor device, and more particularly, to an apparatus for manufacturing a sensor device used for collecting biological information data, and a method for manufacturing a sensor device used for collecting biological information data.

### Description of the Related Art

After entering the modernized era, the public interest in health has been gradually increasing, and in accordance with such a trend, research and development of various devices having a function of collecting biological information data have been actively carried out. Electronic devices such as smartphones, smart watches, arm bands, smart shoes, and smart clothing, which are provided with a function of collecting biological information data, have been released and distributed in the market, and these electronic devices may be utilized to collect biological information data independently or in conjunction with other electronic devices.

In addition to the above-mentioned general-purpose electronic devices, research and development of sensor devices that are provided to be attached to the skin of a human body in order to collect biological information data for the management and treatment of specific diseases have also been actively conducted. Such sensor devices are required not only to ensure the accuracy of biological information data measurement for the effective management and treatment of diseases, but also to secure ease of use, economic feasibility, and the like for general-purpose use. In the case of a transcutaneous sensor device in which at least one end of a sensor member is inserted into a transcutaneous position under the skin of the human body to collect biological information data, there are advantageous aspects in securing the accuracy of biological information data measurement, ease of use, economic feasibility, and the like, and therefore such transcutaneous sensor devices have been receiving great attention as next-generation sensor devices capable of replacing conventional sensor devices.

The transcutaneous sensor device includes a transcutaneous sensor member, at least one end of which is intended to be inserted into a transcutaneous position under the skin, and the transcutaneous sensor member is generally manufactured in the form of a thin plate, pin, or the like to enable smooth subcutaneous insertion. Due to such structural characteristics of the transcutaneous sensor member, breakage or damage of the transcutaneous sensor member may occur during the manufacturing of the transcutaneous sensor device. In order to secure the productivity and economic feasibility of the transcutaneous sensor device, breakage or damage of the transcutaneous sensor member occurring during manufacturing of the transcutaneous sensor device should be actively prevented; however, since no alternative structure of the transcutaneous sensor member has yet been proposed, no practical alternative capable of preventing the breakage or damage of the transcutaneous sensor member during manufacturing of the transcutaneous sensor device has been presented.

Meanwhile, in order to secure the productivity and economic feasibility of the transcutaneous sensor device, it is desirable to manufacture the transcutaneous sensor device using an automation system, but even in this case, a practical alternative has not yet been proposed for an automation system that can effectively prevent the breakage or damage of the transcutaneous sensor member occurring during a manufacturing process of the transcutaneous sensor device, while correctly positioning and assembling the component parts including the transcutaneous sensor member in predetermined positions.

### SUMMARY OF THE INVENTION

One aspect of the present invention is directed to providing a manufacturing apparatus for a sensor device and a manufacturing method for a sensor device capable of effectively preventing the occurrence of manufacturing defects during manufacturing of the sensor device.

Another aspect of the present invention is directed to providing a manufacturing apparatus for a sensor device and a manufacturing method for a sensor device capable of effectively improving workability, productivity, and economic feasibility during manufacturing of the sensor device.

The object of the present invention is not limited to the above description. A person with ordinary skill in the art to which the present invention pertains will have no difficulty in readily understanding additional objects of the present invention from the overall contents of the present specification.

A manufacturing apparatus for a sensor device, according to one aspect of the present invention, may comprise: a component supplying part in which components of a sensor device are disposed; a sensor device assembling part in which the sensor device is assembled by receiving the component transferred from the component supplying part; an operating part including a terminal end portion having a suction hole formed thereon for vacuum suction, the terminal end portion configured to transfer at least one of the components to the sensor device assembling part by vacuum suction; and a control part configured to control an operation of the operating part.

The sensor device assembling part may comprise: a sensor device assembly table on which the sensor device in an assembly stage is disposed; and a position sensor disposed at one side of the sensor device assembly table and configured to detect position information of at least one of the components transferred to the sensor device assembly table, and the control part may control the operating part to adjust a position of at least one terminal end portion based on the position information.

The position sensor may comprise a camera capable of image capturing, and the control part may control the operating part based on image information captured by the camera.

The component may comprise: an intermediate assembly including a housing forming an external shape of the sensor device; and a plate-shaped sensor member disposed on the housing such that a tip end thereof is inserted into a transcutaneous position of a human body to detect biological information.

The terminal end portion may comprise a sensor member gripper configured to grip the sensor member by vacuum suction and move the sensor member to the sensor device assembling part, and the sensor member gripper may transfer the sensor member to the intermediate assembly disposed in the sensor device assembling part.

The sensor member gripper may comprise a sensor member gripper body having, at a tip end portion thereof, a sensor member gripping part recessedly formed in a shape corresponding to at least a part of the sensor member, and one surface of the gripper body forming the sensor member gripping part may be provided with a plurality of sensor member suction holes provided to suction external air.

The sensor member may comprise: a plate-shaped sensor body part disposed on the housing; and a sensor probe part extending from one end of the sensor body part and having a tip end portion disposed outside the housing, and the sensor member gripper may rotationally move during a process of inserting the sensor member into the intermediate assembly such that the sensor body part and the sensor probe part are disposed on different planes.

The terminal end portion may further comprise a housing gripper capable of transferring the intermediate assembly to the sensor device assembling part while moving in a state of gripping the intermediate assembly.

The component supplying part may comprise an intermediate assembly tray in which the intermediate assembly and the sensor member are disposed in pairs, and the terminal end portion may transfer the intermediate assembly and the sensor member disposed in one intermediate assembly tray to the sensor device assembling part to assemble the sensor device.

The component may further comprise a needle separably coupled to the housing from the housing to assist insertion of a tip end of the sensor member into the transcutaneous position of the human body, and the terminal end portion may further comprise a needle gripper configured to transfer the needle to the intermediate assembly disposed in the sensor device assembling part while moving in a state of gripping the needle.

A manufacturing method for a sensor device, according to one aspect of the present invention may comprise: transferring a component from a component supplying part, in which components of a sensor device are disposed, to a sensor device assembling part in which the sensor device is assembled so as to assemble the sensor device, in which at least one of the components may be transferred to the sensor device assembling part by a vacuum suction method, and the sensor device may be assembled.

The component may comprise: an intermediate assembly including a housing forming an external shape of the sensor device; and a plate-shaped sensor member disposed on the housing such that a tip end thereof is inserted into a transcutaneous position of a human body to detect biological information, and the manufacturing method may comprise: transferring the sensor member to the intermediate assembly disposed in the sensor device assembling part by the vacuum suction method to assemble the sensor device.

According to one aspect of the present invention, there may be provided a manufacturing apparatus for a sensor device and a manufacturing method for a sensor device capable of effectively preventing the occurrence of manufacturing defects during manufacturing of the sensor device.

Another aspect of the present invention may provide a manufacturing apparatus for a sensor device and a manufacturing method for a sensor device capable of effectively improving workability, productivity, and economic feasibility during manufacturing of the sensor device.

The effects of the present invention are not limited to the above description, and may include matters that can be reasonably inferred from the following description by a person with ordinary skill in the art to which the present invention pertains.

### BRIEF DESCRIPTION OF THE DRAWINGS

FIG. 1 is a perspective view exemplarily showing a transcutaneous sensor device to which needles are coupled.
FIG. 2 is an exploded perspective view exemplarily illustrating the transcutaneous sensor device.
FIGS. 3 to 5 are assembly perspective views sequentially illustrating an example of an assembly process of the transcutaneous sensor device.
FIG. 6 is a perspective view illustrating an exemplary embodiment of a manufacturing apparatus for a sensor device.
FIG. 7 is a perspective view enlargedly illustrating a first robot arm hand part illustrated in FIG. 6.
FIG. 8 is a perspective view enlargedly illustrating a sensor member gripper illustrated in FIG. 7.
FIG. 9 is a perspective view enlargedly illustrating a second robot arm hand part illustrated in FIG. 6.
FIGS. 10 and 11 are a perspective view and a plan view enlargedly illustrating a sensor device assembly table illustrated in FIG. 6.
FIG. 12 is a perspective view enlargedly illustrating a sensor device assembling part illustrated in FIG. 6.
FIG. 13 is a plan view enlargedly illustrating a component supplying part illustrated in FIG. 6.
FIGS. 14 to 19 are drawings sequentially illustrating a process of moving an intermediate assembly by a housing gripper.
FIGS. 20 to 25 are drawings sequentially illustrating a process of removing a release paper by a release paper removing unit.
FIGS. 26 to 34 are drawings sequentially illustrating processes of moving and assembling needles by a needle gripper.
FIGS. 35 to 43 are drawings sequentially illustrating processes of moving and assembling a sensor member by a sensor member gripper.
FIGS. 44 to 48 are drawings enlargedly and sequentially illustrating operations of the sensor member gripper during assembly of the sensor member.
FIGS. 49 and 50 are drawings sequentially illustrating a process in which a button cover gripper grips a button cover.
FIGS. 51(a) to 51(c) are drawings sequentially illustrating a process in which the button cover is transferred from a button cover supplying table to a button cover gripper.
FIGS. 52 to 56 are drawings sequentially illustrating processes of moving and assembling a button member by a button member gripper.
FIGS. 57 to 63 are drawings sequentially illustrating a process of attaching a button cover by a button cover attaching unit.

### DETAILED DESCRIPTION OF THE INVENTION

Hereinafter, preferred embodiments of a manufacturing apparatus for a sensor device and a manufacturing method for a sensor device according to one aspect of the present invention will be described in more detail with reference to the accompanying drawings. Embodiments of the present invention may be modified into various forms, and the scope of the present invention should not be construed as being limited to the embodiments described below. These embodiments are provided to more specifically describe the present invention to those skilled in the technical field to which the present invention pertains. Accordingly, a shape of each element illustrated in the drawings may be emphasized or exaggerated for a clearer description.

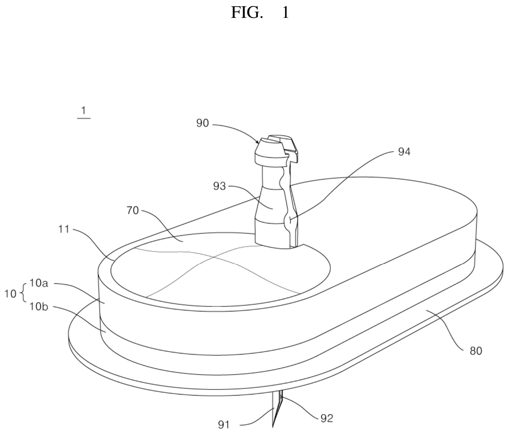

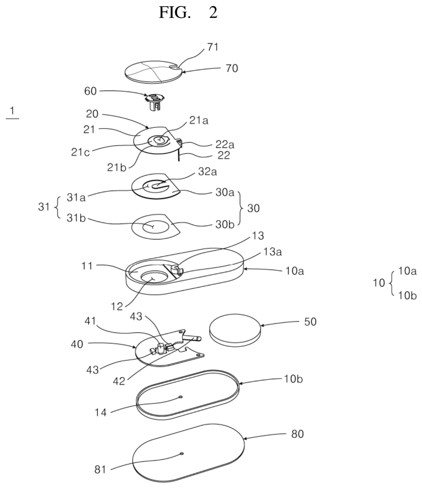

Hereinafter, with reference to FIGS. 1 and 2, an example of a sensor device manufactured by a manufacturing apparatus for a sensor device and a manufacturing method for a sensor device according to one aspect of the present invention will be described in more detail. In the following description, a transcutaneous sensor device in which subcutaneous insertion is intended will be described as one example of the sensor device, but the manufacturing apparatus for a sensor device and the manufacturing method for a sensor device according to one aspect of the present invention are not necessarily limited to manufacturing of the transcutaneous sensor device, and may be variously modified and applied as a manufacturing apparatus and a manufacturing method for any sensor device capable of detecting biological information. The biological information detected by the sensor device may be interpreted as including all biological information that can be detected from a human body, and the biological information detected by the sensor device may be a glucose concentration. Meanwhile, the sensor device manufactured by the manufacturing apparatus for a sensor device and the manufacturing method for a sensor device according to one aspect of the present invention may be a sensor module of a continuous glucose monitor (CGM) attachable to a human body to detect blood glucose information for a certain period of time.

As illustrated in FIG. 2, the sensor device 1 may include a housing 10 provided to form an external shape of the sensor device 1, a sensor member 20 provided such that one end portion thereof protrudes outward from one end of the housing 10 for insertion into a transcutaneous position under the human body, an electronic board 40 disposed inside the housing 10 to transmit biological information-related data sensed by the sensor member 20 to an external terminal, a battery 50 provided to supply power to the electronic board 40, and a housing adhesive member 80 disposed at one end of the housing 10, from which one end of the sensor member 20 protrudes, to provide adhesive force so that the housing 10 is attached to the human body.

The sensor member 20 may include a sensor body part 21 provided in a plate shape, and a sensor probe part 22 extending from one end of the sensor body part 21 so that a tip end portion thereof is inserted into a transcutaneous position under the skin. In a central region of the sensor body part 21, a sensor body part through-hole 21a penetrating the sensor body part 21 may be provided, and an incision part 21b may be formed at a circumferential portion of the sensor body part through-hole 21a to incise a partial region of the sensor body part 21. The incision part 21b may be formed in a spiral shape, and a sensor contact part 21c may be separated from the sensor body part 21 by the incision part 21b formed at the circumferential portion of the sensor body part through-hole 21a. As a button pressing part 61 of a button member 60, which will be described later, presses the sensor contact part 21c, the shape of a partial region of the sensor body part 21 having the incision part 21b may be deformed, and accordingly, the sensor contact part 21c may be electrically brought into contact with an electronic board contact part 43 provided on the electronic board 40.

Between the sensor body part 21 and the sensor probe part 22, an extension part 22a connecting the sensor body part 21 and the sensor probe part 22 may be provided. The extension part 22a may include a first extension part (reference numeral not assigned) formed to extend from one end of the sensor body part 21 along a direction generally parallel to the sensor probe part 22, and a second extension part (reference numeral not assigned) formed to extend from one end of the first extension part along a direction intersecting the sensor probe part 22 and connected to the sensor probe part 22. During assembly of the sensor device 1, the sensor member 20 may be coupled to the housing 10 while the first extension part is bent, and in the assembled sensor device 1, since the first extension part maintains the bent state, the sensor body part 21 and the sensor probe part 22 may be disposed on different planes.

The housing 10 may include a first housing 10a and a second housing 10b provided to be mutually couplable. The electronic board 40 and the battery 50 may be accommodated in an internal space of the housing 10 formed as the first housing 10a and the second housing 10b are coupled. On one surface of the first housing 10a, a sensor member seating part 11 recessed from one surface of the first housing 10a toward a central portion thereof may be provided. The sensor member seating part 11 may be recessedly formed on the one surface of the first housing 10a in a shape corresponding to the sensor body part 21, and the sensor body part 21 may be disposed to be accommodated in the sensor member seating part 11 during an assembly process of the sensor device 1. A housing opening 12, which is formed in a shape penetrating the first housing 10a in a thickness direction at a central portion of the sensor member seating part 11, may be provided. A sensor contact part 21c of a sensor body part 21 may be electrically contacted to an electronic board contact part 43 of an electronic board 40 disposed inside a housing 10 through the housing opening 12. At one side of the sensor member seating part 11, a first housing through-hole 13, into which a sensor probe part 22 may be inserted to penetrate, may be formed to penetrate the first housing 10a. At one side of the first housing through-hole 13, an extension slit 13a, through which an extension part 22a may be disposed to penetrate, may be formed to penetrate the first housing 10a. The extension slit 13a may be provided in a shape extending from one end of the first housing through-hole 13.

A second housing through-hole 14 may be formed to penetrate a second housing 10b along the thickness direction at a position corresponding to the first housing through-hole 13. A sensor probe part 22 and a needle body 91, which are inserted to penetrate through the first housing through-hole 13, may be disposed such that tip end portions are positioned outside the housing 10 by penetrating the second housing through-hole 14, and the needle body 91 may be separated from a sensor device 1 by being discharged through the first housing through-hole 13 and the second housing through-hole 14 after a tip end of the sensor probe part 22 is inserted and disposed at a transcutaneous position under the skin.

A sensor adhesive member 30 may be disposed between the sensor body part 21 and the sensor member seating part 11 so as to fix the sensor member 20 to the first housing 10a. The sensor adhesive member 30 may include an adhesive sheet 30b having adhesives on both surfaces and a release paper 30a provided to protect one surface of the adhesive sheet 30b. An intermediate assembly 1' of the sensor device 1, which will be described later, may be provided in a state in which the adhesive sheet 30b, from which the release paper 30a is not separated, is attached to the sensor member seating part 11. A release paper through-hole 31a and an adhesive sheet through-hole 31b, which penetrate the release paper 30a and the adhesive sheet 30b in the thickness direction, may be respectively formed at central portions of the release paper 30a and the adhesive sheet 30b. The release paper through-hole 31a and the adhesive sheet through-hole 31b may be respectively formed to penetrate the release paper 30a and the adhesive sheet 30b in corresponding shapes to each other. A release paper gripping part 32a may be provided in a shape extending from an inner end of a release paper 30a, which forms a release paper through-hole 31a, toward a central portion of the release paper through-hole 31a. During a manufacturing process of a sensor device 1, a sensor adhesive member 30 may be provided in a state of being attached to a sensor member seating part 11. An operator may grip the release paper gripping part 32a, remove the release paper 30a from the adhesive sheet 30b, and then closely attach one surface of the sensor body part 21 to the sensor member seating part 11, to which the adhesive sheet 30b is attached, thereby coupling a housing 10 and a sensor member 20. A coupling force between the housing 10 and the sensor member 20 may be enhanced by the adhesive sheet 30b disposed between the sensor body part 21 and the sensor member seating part 11. In addition, the adhesive sheet 30b disposed between the sensor body part 21 and the sensor member seating part 11 may effectively prevent external contaminants or moisture from being introduced to an interior of the housing 10 through the housing opening 12.

An electronic board 40 may include an electronic board contact part 43 configured to be electrically connected to a sensor contact part 21c, a transmit module (not illustrated) capable of transmitting biological information data detected from the sensor member 20 to the outside, a button guide part 41, which protrudes in a shape to guide a movement direction when a button member 60 is pressed and moved, and a contact terminal 42 configured to be electrically connected to a battery 50. The electronic board 40 may be disposed inside the housing 10 such that the electronic board contact part 43 and the button guide part 41 are positioned at positions corresponding to the housing opening 12. Although a coin-type battery 50 is illustrated in FIG. 2, the battery 50 provided in the sensor device 1 is not necessarily limited to the coin-type battery 50, and various modifications may be applied as long as they are means capable of supplying power to the electronic board 40.

The button member 60 may include a button pressing part 61 disposed along a direction substantially parallel to the sensor body part 21, and a button body part 62, which extends from one end of the button pressing part 61 facing the sensor body part 21 toward the electronic board 40. At a tip end of the button body part 62, a guide groove (not illustrated) having a shape corresponding to the button guide part 41 may be provided.

While one end of the button guide part 41 is partially inserted into the guide groove (not illustrated), when the button pressing part 61 is pressed, the button pressing part 61 may move along an extending direction of the button guide part 41. As a user presses the button member 60, the sensor contact part 21c may be pressed at a side end of the button pressing part 61 so that its shape is deformed, and as the deformed sensor contact part 21c comes into contact with the electronic board contact part 43, the sensor member 20 and the electronic board 40 may be electrically connected.

A button cover 70 may be disposed on the sensor member seating part 11 so as to block the sensor member seating part 11 from the outside. In the sensor member seating part 11, the sensor member 20, the adhesive sheet 30b, and the button member 60 may be sequentially disposed, and the button cover 70 may be disposed on the sensor member seating part 11 so as to block external exposure of the sensor member 20, the adhesive sheet 30b, and the button member 60. In order to allow flexible deformation of a button cover 70 when the button member 60 is pressed by a user, the button cover 70 may preferably be made of a flexible material such as rubber or silicone. At one end of the button cover 70 facing the sensor member seating part 11, an additional adhesive sheet (not illustrated) having adhesives on both surfaces may be provided. By the adhesive sheet (not illustrated) disposed at one end of the button cover 70, the button cover 70 may be more firmly fixed to the sensor member seating part 11, and the introduction of external contaminants or moisture toward the sensor body part 21 may also be effectively prevented. The adhesive sheet (not illustrated) disposed at one end of the button cover 70 may have a penetration part formed to penetrate through a thickness direction at a central portion, such that the adhesive sheet (not illustrated) disposed at one end of the button cover 70 is not directly attached to the button member 60, but may be attached to one surface of the sensor body part 21 or to one surface of the first housing 10 forming the sensor member seating part 11. At one side end of the button cover 70, a button cover incision part 71 may be provided in a shape recessed toward a central portion from a side end portion of the button cover 70, in order to avoid interference with a needle 90 during assembly of the sensor device 1.

A housing adhesive member 80 may be disposed at one end of the housing 10, from which a sensor probe part 22 protrudes outward. The sensor device 1 may maintain an attached state to human skin by the housing adhesive member 80. The housing adhesive member 80 may include a housing adhesive member through-hole 82 formed to penetrate the housing adhesive member 80 along a thickness direction at a position corresponding to a second housing through-hole 14, and tip ends of the sensor probe part 22 and a needle body 91 may protrude outward from the housing 10 through the housing adhesive member through-hole 82. The housing adhesive member 80 may have a first attachment surface attached to one end surface of the housing 10, and a second attachment surface attached to human skin, and a release paper (not illustrated) for preventing contamination of the second attachment surface may be further provided on the second attachment surface. Meanwhile, although the drawing illustrates the housing adhesive member 80 having a larger area than that of one end portion of the housing 10, the shape of the housing adhesive member 80 is not necessarily limited thereto, and various modifications may be applied as long as the shape and material can improve the ease of use of the user while the sensor device 1 is fixed to the human body.

A needle 90 is a component provided to pierce the epidermis of the human body so that a tip end portion of a sensor probe part 22 is disposed at a transcutaneous position under the skin of the human body. The needle 90 may be assembled together with the sensor device 1 during a manufacturing process of the sensor device 1, but after the sensor device 1 is attached to the human body, the needle 90 may be separated and discharged from the sensor device 1. The needle 90 may include a needle body 91 having a pointed tip to pierce the epidermis of the human body, and a needle holder 93 configured to fix the needle body 91. At a side end of the needle body 91, a needle body incision part 92 formed to extend along a longitudinal direction of the needle body 91 may be provided, and in the needle holder 93, a needle holder incision part 94 formed to extend from a tip end of the needle holder 93 toward a side end of the needle holder 93 along a longitudinal direction of the needle holder 93 may be provided. The needle body incision part 92 and the needle holder incision part 94 may be connected to each other to form a shape extending along the longitudinal direction of the needle 90. The needle body incision part 92 and the needle holder incision part 94 may provide a path through which a sensor probe part 22 and an extension part 22a move during an assembly process of a sensor device 1 or during an attachment process of the sensor device 1 to the human body.

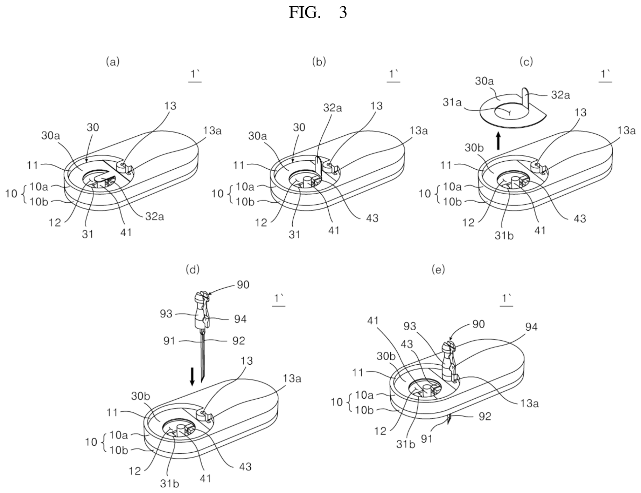

Hereinafter, the assembly process of the sensor device will be described in more detail with reference to FIGS. 3 to 5.

FIG. 3(a) is a perspective view exemplarily illustrating an intermediate assembly 1' of the sensor device 1. As illustrated in FIG. 3(a), an intermediate assembly 1', in which an electronic board 40 and a battery 50 are disposed inside a housing 10 and a sensor adhesive member 30 is attached to a sensor member seating part 11, may be provided. The intermediate assembly 1' and the sensor device 1 may be assembled and manufactured through a manual process by an operator or an automated process using mechanical equipment; however, for convenience of explanation, a case in which the sensor device 1 is assembled by manual operation of the operator will be described as an example of the assembly process of the sensor device 1.

As illustrated in FIGS. 3(b) and 3(c), the operator may bend and deform a release paper gripping part 32a so that a tip end portion of the release paper gripping part 32a is positioned outside a housing opening 12, and then remove the release paper 30a from the sensor member seating part 11 by pulling the release paper gripping part 32a using a hand tool such as tweezers. When the release paper 30a is removed from the sensor member seating part 11, the adhesive sheet 30b may maintain a state of being adhered to the sensor member seating part 11.

Thereafter, as illustrated in FIGS. 3(d) and 3(e), the operator may couple a needle 90 to the intermediate assembly 1' from which the release paper 30a has been removed. A needle body 91 may be disposed to penetrate the first housing through-hole 13 and the second housing through-hole 14, and the needle body 91 penetrating the second housing through-hole 14 may be disposed such that a tip end portion of the needle body 91 protrudes outward from one end portion side of the second housing 10b. A needle holder 93 may be disposed such that a needle holder incision part 94 is connected to an extension slit 13a, and, as described later, the extension part 22a may pass through the needle holder incision part 94 and be introduced into the extension slit 13a. Meanwhile, although the above description exemplarily describes coupling of the needle 90 to the housing 10 after removal of the release paper 30a, the release paper 30a may be removed after the needle 90 is coupled to the housing 10.

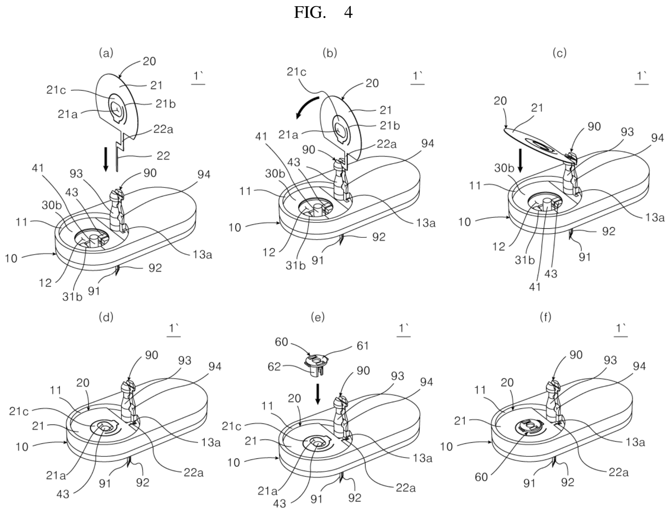

As illustrated in FIGS. 4(a) and 4(b), the operator may grip the sensor member 20 by hand tool or fingers and introduce portions of the sensor probe part 22 and the extension part 22a into the needle holder incision part 94. When a tip end portion of the sensor probe part 22 is not precisely positioned at a tip end portion of the needle holder incision part 94, the protective layer of the sensor probe part 22 may be damaged, or the sensor probe part 22 may be bent or broken during the introduction process of the sensor probe part 22. Meanwhile, the sensor probe part 22 should be introduced into an inside of the needle holder incision part 94 while maintaining a state spaced apart at a certain distance from both inner walls forming the needle holder 93, which forms the needle holder incision part 94, but depending on a work environment or work condition, a case may occur where a spacing distance between the sensor probe part 22 and the inner wall of the needle holder incision part 94 is not uniformly maintained. In this case, the sensor probe part 22 may come into contact with the inner wall of the needle holder incision part 94, and the protective layer of the sensor probe part 22 may be damaged, or the sensor probe part 22 may be bent or broken.

As illustrated in FIGS. 4(c) and 4(d), after an extension part 22a (preferably a second extension part) is introduced into an interior of a needle holder incision part 94, an operation of pressing the sensor body part 21 toward a sensor member seating part 11 to bend the extension part 22a is performed. As the extension part 22a is bent, the sensor body part 21 is placed in a direction substantially parallel to an adhesive sheet 30b disposed on the sensor member seating part 11, and as the sensor body part 21 is pressed toward the adhesive sheet 30b, the sensor body part 21 is adhesively fixed by the adhesive sheet 30b. When the sensor body part 21 is pressed in a state in which the extension part 22a is not introduced into the interior of the needle holder incision part 94, or when a force of a critical point or more is momentarily applied during a process of pressing the sensor body part 21, damage, bending, or breakage of a sensor probe part 22 or the extension part 22a may occur. In a state in which one surface of the sensor body part 21 is attached to the adhesive sheet 30b, the sensor probe part 22 is disposed inside a needle body 91, and during separation and discharge of the needle 90, contact or interference of the needle body 91 with the sensor probe part 22 and the extension part 22a may be eliminated by the needle body incision part 92 formed in the needle body 91.

As illustrated in FIGS. 4(e) and 4(f), after attaching one surface of the sensor body part 21 to the adhesive sheet 30b, the operator may dispose a button member 60 into the sensor body part through-hole 21a. A button body part 62 may be disposed to maintain a state in which one end thereof is inserted into the sensor body part through-hole 21a, and a tip end portion of a button guide part 41 may be disposed to be positioned inside a guide groove (not illustrated) formed in the button body part 62. A button pressing part 61 may be disposed such that its side end is in close contact with or spaced at a predetermined interval from a sensor contact part 21c.

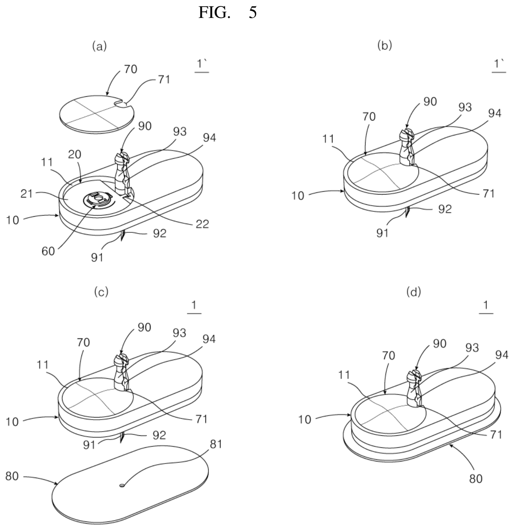

As illustrated in FIGS. 5(a) and 5(b), after disposing the button member 60, the operator may dispose a button cover 70 on the sensor member seating part 11. As described above, an adhesive sheet (not illustrated) having an adhesive on one surface may be provided on one surface of the button cover 70 facing the sensor member seating part 11, and detachment of the button cover 70 from the sensor member seating part 11 may be prevented by the adhesive sheet (not illustrated). During a process of disposing the button cover 70, the operator may allow the needle holder 93 to be disposed at the button cover incision part 71 and to pass through the button cover incision part 71, thereby minimizing interference of the needle holder 93 with the button cover 70.

As illustrated in FIGS. 5(c) and 5(d), after disposing the button cover 70, the operator may dispose a housing adhesive member 80 on one end of the housing 10, from which a tip end of a needle body 91 protrudes. The protruding tip end of the needle body 91 may be disposed to penetrate a housing adhesive member through-hole 82 formed through the housing adhesive member 80. Meanwhile, although the above description exemplarily describes a case in which the housing adhesive member 80 is attached to one end of the housing 10 after disposing the button cover 70, it may include cases where the housing adhesive member 80 is disposed at a stage of the intermediate assembly 1' as shown in FIG. 3(a) or disposed at a subsequent stage thereafter.

The inventors of the present invention have conducted in-depth research on a manufacturing apparatus and a manufacturing method capable of effectively preventing the occurrence of manufacturing defects during the manufacture of the sensor device 1, while effectively improving productivity and economic feasibility, and as a result, have derived a manufacturing apparatus for a sensor device and a method of manufacturing the sensor device, which will be described later.

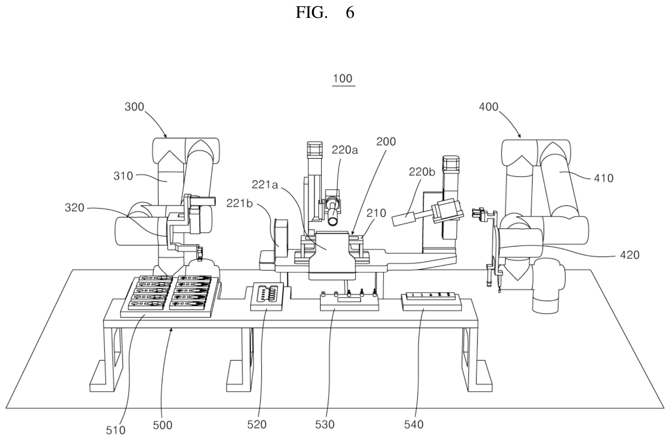

Hereinafter, with reference to FIGS. 6 to 13, a manufacturing apparatus for a sensor device according to one aspect of the present invention will be described in more detail.

As illustrated in FIG. 6, a sensor device manufacturing apparatus 100 according to one aspect of the present invention may include: sensor device assembling part 200, in which assembly of a sensor device 1 is performed; a component supplying part 500, in which components used for assembly of the sensor device 1 are disposed; and an operating part provided so as to sequentially transfer the components constituting the sensor device 1 from the component supplying part 500 to the sensor device assembling part 200 for assembly. The operating part may be robot arms 300 and 400. Hereinafter, a sensor device manufacturing apparatus 100 including two robot arms 300 and 400 will be exemplarily described. However, the number and position of the robot arms provided in the sensor device manufacturing apparatus 100 according to one aspect of the present invention are not necessarily limited thereto, and the number and position of the robot arms may be variously modified and applied depending on the work environment and work conditions of the sensor device 1 assembly process.

A first robot arm 300 may include a first robot arm joint part 310 including a plurality of links and joints, and a first robot arm hand part 320 provided at one end of the first robot arm joint part 310. The first robot arm hand part 320 may include a first terminal end portion capable of transferring components constituting the sensor device 1 to predetermined positions, and the first terminal end portion may be one of first end effectors 340, 350, and 360. A second robot arm 400 may include a second robot arm joint part 410 including a plurality of links and joints, and a second robot arm hand part 420 provided at one end of the second robot arm joint part 410. The second robot arm hand part 420 may include a second terminal end portion capable of transferring components constituting the sensor device 1 to predetermined positions, and the second terminal end portion may be one of second end effectors 440, 450, and 460. Hereinafter, as an example, a case will be described in which a housing gripper 340, a sensor member gripper 350, and a release paper removing unit 360 are provided as the first end effectors of the first robot arm hand part 320, and a needle gripper 440, a button cover attaching unit 450, and a button member gripper 460 are provided as the second end effectors of the second robot arm hand part 420. However, the configuration of the end effectors provided in the sensor device manufacturing apparatus 100 according to one aspect of the present invention is not necessarily limited thereto, and the configuration of the end effectors may be variously modified and applied depending on the work environment and work conditions of the sensor device 1 assembly operation.

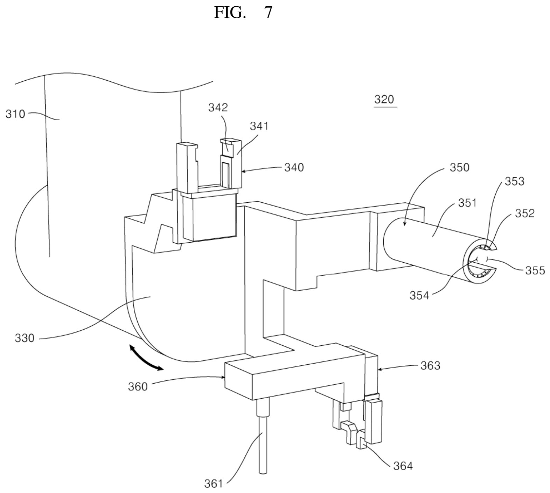

As illustrated in FIG. 7, a first rotation plate 330 may be shaft-coupled to one end of the first robot arm joint part 310 so as to be rotatably driven, and the first robot arm 300 may be provided with a first rotation plate driving part (not illustrated) configured to provide rotational driving force to the first rotation plate 330. The housing gripper 340, the sensor member gripper 350, and the release paper removing unit 360 may be disposed on a circumferential portion of the first rotation plate 330.

The housing gripper 340 may include housing gripper tongs 341 provided to grip an intermediate assembly 1' disposed in a component supplying part 500 and transfer it to a sensor device assembling part 200, and a housing gripper tong driving part (not illustrated) provided to transfer a driving force to the housing gripper tong 341. On facing inner surfaces of the housing gripper tongs 341, housing seating parts 342 may be recessedly formed in a symmetrical shape, and during a process in which the housing gripper tongs 341 grip the intermediate assembly 1'from the component supplying part 500 and transfers it to the sensor device assembling part 200, both side ends of the intermediate assembly 1' may be disposed in the housing seating parts 342, thereby more effectively improving gripping stability of the intermediate assembly 1'. The housing gripper 340 may not only transfer the intermediate assembly 1' to the sensor device assembling part 200, but also discharge the sensor device 1, which has been completely assembled in the sensor device assembling part 200, from the sensor device assembling part 200. Meanwhile, although the above description exemplarily describes the housing gripper 340 that grips and transfers the intermediate assembly 1' or the sensor device 1 by a tong-type gripping method, the gripping method of the intermediate assembly 1' or the sensor device 1 by the housing gripper 340 is not necessarily limited thereto, and the housing gripper 340 may grip and transfer the intermediate assembly 1' or the sensor device 1 by various methods such as a lifting method or a vacuum suction method.

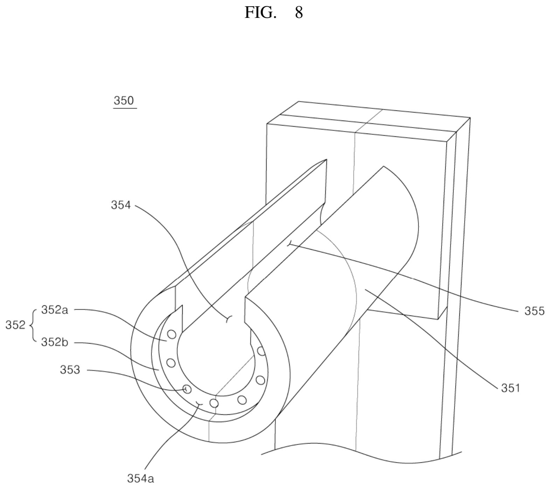

The sensor member gripper 350 may be disposed on the first rotation plate 330 so as to grip a sensor member 20 disposed in the component supplying part 500 and transfer it to the intermediate assembly 1' seated on the sensor device assembling part 200. As illustrated in FIG. 8, the sensor member gripper 350 may include a cylindrical sensor member gripper body 351, and in the sensor member gripper body 351, a sensor member gripper internal space 354 connected to the outside at a tip end portion may be formed to extend along a longitudinal direction of the sensor member gripper body 351. A sensor member gripper incision part 355 may be provided in a shape in which the sensor member gripper body 351 is incised along the longitudinal direction of the sensor member gripper body 351 from one side end of the sensor member gripper body 351, so as to connect the sensor member gripper internal space 354 with the outside. In a case where the sensor member gripper 350 suctions and moves the sensor member 20 for assembly of the sensor device 1, a needle 90 provided in the intermediate assembly 1' may be disposed in the sensor member gripper internal space 354 or the sensor member gripper incision part 355, thereby effectively avoiding movement interference of the sensor member gripper 350 caused by the needle 90 provided in the intermediate assembly 1'. At a tip end of the sensor member gripper body 351, a sensor member gripping part 352 recessedly formed in a shape corresponding to a shape of the sensor body part 21 may be provided. The sensor member gripping part 352 may include a sensor member seating surface 352a formed to be recessed from a tip end portion of the sensor member gripper body 351 by a predetermined distance, and a sensor member gripping part boundary surface 352b provided along a circumferential portion of the sensor member seating surface 352a. At a tip end portion of a sensor member gripper body 351, a sensor member seating space 354a partitioned by a sensor member seating surface 352a and a sensor member gripping part boundary surface 352b may be provided, and during movement of a sensor member 20 by the sensor member gripper 350, the sensor body part 21 may maintain a state of being accommodated in the sensor member seating space 354a. The sensor member seating space 354a may be provided at a tip end portion side of a gripper internal space 354 so as to be connected with the gripper internal space 354. On the sensor member seating surface 352a, a plurality of sensor member suction holes 353 for suctioning air for suction of the sensor member 20 may be disposed spaced apart from each other at predetermined intervals along a circumferential direction of the sensor member gripping part 352. Since the plurality of sensor member suction holes 353 are disposed at positions recessed by a predetermined distance from the tip end portion of the sensor member gripper body 351, and the sensor member seating surface 352a on which the sensor member suction holes 353 are formed has a shape surrounded by the sensor member gripping part boundary surface 352b, a vacuum suction force generated by the sensor member suction holes 353 may be improved. The sensor device manufacturing apparatus 100 may further include a vacuum pump (not illustrated) for providing suction force when the sensor member 20 is suctioned and moved, and a vacuum pipe (not illustrated) connecting the vacuum pump and the sensor member suction holes 353.

During a process of transferring the sensor member 20 from the component supplying part 500 to the sensor device assembling part 200, the sensor body part 21 may be disposed on the sensor member gripping part 352, and the sensor probe part 22 and/or the extension part 22a may be disposed at a tip end portion of a sensor member gripper incision part 355, and as the sensor member suction holes 353 maintain a state of suctioning air, the sensor member 20 may maintain a state of being stably seated on the sensor member gripper 350 by vacuum suction. Since the sensor member gripper 350 grips the sensor member 20 by a vacuum suction method and moves while assembling the sensor device **1,** surface damage of the sensor member 20 that may occur during gripping and movement processes of the sensor member 20 may be more effectively prevented. The sensor member gripper 350 moves toward the sensor device assembling part 200 while maintaining a state in which the sensor body part 21 is accommodated in the sensor member seating space 354a, thereby more effectively preventing the sensor member 20 from being broken or damaged by external impact during movement of the sensor member 20 by the sensor member gripper 350. In addition, since the sensor member gripper 350 performs an operation of inserting the sensor probe part 22 into a first housing through-hole 13 or bending the extension part 22a while the sensor body part 21 is accommodated in the sensor member seating space 354a, during a process of disposing the sensor member 20 on the intermediate assembly 1', detachment of the sensor member 20 from the sensor member gripper 350 or breakage or damage of the sensor member 20 due to pressing of a specific region of the sensor member 20 with a force of a critical point or more may be effectively prevented.

A release paper removing unit 360 may be disposed on the first rotation plate 330 so as to remove a release paper 30a from an intermediate assembly 1' seated on the sensor device assembling part 200. The release paper removing unit 360 may include a release paper suction rod 361 configured to pull a tip end portion of a release paper gripping part 32a outward from a release paper through-hole 31a so that the tip end portion of the release paper gripping part 32a is discharged from the release paper through-hole 31a, and a release paper gripper 363 configured to move while gripping the release paper gripping part 32a, which has its tip end portion discharged outward, to remove the release paper 30a from the sensor member seating part 11. At a tip end of the release paper suction rod 361, a release paper suction hole 362 configured to suction air for suction of the release paper gripping part 32a may be provided. For effective vacuum suction of the release paper gripping part 32a, a release paper suction hole 362 may be provided to have a diameter larger than a width of the release paper gripping part 32a. Meanwhile, in order to eliminate operation interference caused by a release paper gripper 363, which will be described later, during vacuum suction of the release paper gripping part 32a, it may be preferable that a release paper suction rod 361 is provided such that the release paper suction hole 362 is disposed at a position more protruded than the release paper gripper 363. The sensor device manufacturing apparatus 100 may further include a vacuum pump (not illustrated) for providing suction force when the release paper gripping part 32a is suctioned, and a vacuum pipe (not illustrated) connecting the vacuum pump and the release paper suction hole 362. The release paper suction rod 361 may move to a position where the release paper suction hole 362 can be closely contacted with a tip end portion of the release paper gripping part 32a, and as air is suctioned through the release paper suction hole 362, the release paper gripping part 32a may be vacuum-suctioned to the tip end portion of the release paper suction rod 361. As the release paper suction rod 361 moves in a state in which the tip end portion of the release paper gripping part 32a is vacuum-suctioned to the tip end portion of the release paper suction rod 361, the tip end portion of the release paper gripping part 32a may move along a movement direction of the release paper suction rod 361. As the tip end portion of the release paper gripping part 32a moves, the release paper gripping part 32a may be switched into a folded state, and accordingly, the tip end portion of the release paper gripping part 32a may be discharged outward from the release paper through-hole 31a. The release paper gripper 363 may include release paper gripper tongs 364 configured to grip the release paper gripping part 32a, and a release paper gripper driving part (not illustrated) provided to transfer a driving force to the release paper gripper tongs 364. The release paper gripper tongs 364 may grip the tip end portion of the release paper gripping part 32a, which is discharged from the release paper through-hole 31a, and may move in such a state so that the release paper 30a is separated and discharged from the sensor member seating part 11. The discharge of the tip end portion of the release paper gripping part 32a from the release paper through-hole 31a and the separation and discharge of the release paper 30a from the sensor member seating part 11 may not be separately performed, and may include a case in which the release paper 30a is separated and discharged from the sensor member seating part 11 by any one of the release paper suction rod 361 or the release paper gripper 363. However, in view of the trend that the sensor device 1 is gradually miniaturized for factors such as ease of use, it may be more preferable, in terms of work precision, to sequentially use the release paper suction rod 361 and the release paper gripper 363 to separately perform the discharge of the tip end portion of the release paper gripping part 32a and the separation and discharge of the release paper 30a.

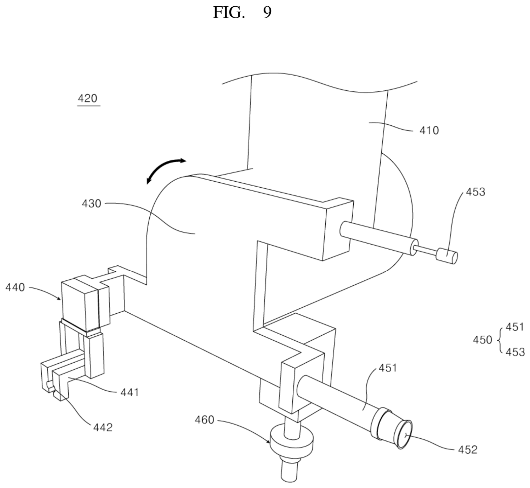

As illustrated in FIG. 9, a second rotation plate 430 may be shaft-coupled to one end of a second robot arm joint part 410 so as to be rotatably driven, and a second rotation plate driving part (not illustrated) configured to provide rotational driving force to the second rotation plate 430 may be provided in the second robot arm 400. A needle gripper 440, a button cover attaching unit 450, and a button member gripper 460 may be disposed on a circumferential portion of the second rotation plate 430.

The needle gripper 440 may include needle gripper tongs 441 configured to grip a needle 90 disposed in a component supplying part 500 and transfer it to an intermediate assembly 1' seated on a sensor device assembling part 200, and a needle gripper tongs driving part (not illustrated) configured to transfer a driving force to the needle gripper tongs 441. On facing inner surfaces of the needle gripper tongs 441, a needle seating part 442 having shapes corresponding to a needle holder 93 may be symmetrically and recessedly formed. During a process in which the needle gripper tongs 441 grip the needle 90 from the component supplying part 500 and move it to the sensor device assembling part 200, both side ends of the needle holder 93 may be disposed or closely contacted in the needle seating part 442, thereby more effectively improving gripping stability of the needle 90. The needle gripper tongs 441 may be provided not only to grip and move the needle 90, but also to press a tip end of the needle holder 93 with closed needle gripper tongs 441 so as to completely push a needle body 91 into a first housing through-hole 13 and a second housing through-hole 14. Meanwhile, although the above description exemplarily describes a needle gripper 440 that grips and transfers the needle 90 by a tong-type gripping method, the gripping method of the needle 90 by the needle gripper 440 is not necessarily limited thereto, and the needle gripper 440 may transfer the needle 90 to the sensor device assembling part 200 by various methods such as a lifting method or a vacuum suction method.

A button cover attaching unit 450 may include a button cover gripper 451 configured to grip a button cover 70 disposed in the component supplying part 500 and transfer it to a sensor member seating part 11 of an intermediate assembly 1' seated on the sensor device assembling part 200, and a button cover presser 453 configured to press the button cover 70 transferred to the sensor member seating part 11 to firmly attach the button cover 70 to the sensor member seating part 11. The button cover gripper 451 may include a button cover suction hole 452 configured to provide suction force for gripping the button cover 70 by vacuum suction. The button cover suction hole 452 provided at a tip end portion of the button cover gripper 451 may have a diameter smaller than that of the button cover 70 for accurate vacuum suction of the button cover 70. The sensor device manufacturing apparatus 100 may further include a vacuum pump (not illustrated) for providing suction force during suctional movement of the button cover 70, and a vacuum pipe (not illustrated) connecting the vacuum pump and the button cover suction hole 452. The button cover presser 453 may move along a circumferential portion of the button cover 70 disposed on the sensor member seating part 11 and may be configured to press the button cover 70, and may further include buffer means (not illustrated) for preventing excessive pressure from being applied to the button cover 70. From the viewpoint of work efficiency, the button cover gripper 451 and the button cover presser 453 may be disposed on the second rotation plate 430 so as to extend along a parallel direction. In order to avoid operation interference between the button cover presser 453 during a transferring process of the button cover 70 by the button cover gripper 451, and operation interference of the button cover gripper 451 during a process of pressing the button cover 70 by the button cover presser 453, the button cover gripper 451 and the button cover presser 453 may be disposed on the second rotation plate 430 so as to be spaced apart from each other by a predetermined distance. Meanwhile, although the button cover gripper 451 may also variously employ methods such as a tongs-type method or a lifting method, a vacuum suction method may be preferably applied in view of preventing an adhesive surface of the button cover 70 from being damaged during a movement process of the button cover 70.

A button member gripper 460 may be disposed on a second rotation plate 430 so as to grip a button member 60 disposed in a component supplying part 500 and transfer it to an intermediate assembly 1' seated on a sensor device assembling part 200. The button member gripper 460 may include a button member suction hole 461 configured to provide suction force for gripping the button member 60 by vacuum suction, and the button member suction hole 461 may be provided at one end of the button member gripper 460 so as to accommodate one end of the button member 60. Inside the button member suction hole 461 or at one end side of the button member 60, a stopper member (reference numeral not assigned) may be provided to assist in preventing the button member 60 from being completely introduced into the interior of the button member suction hole 461. That is, during vacuum suction of the button member 60 by the button member gripper 460, a stopper member (reference numeral not assigned) provided inside the button member suction hole 461 or at one end of the button member 60 may allow the other end portion of the button member 60 to maintain a state of being protruded outward from the button member suction hole 461. The sensor device manufacturing apparatus 100 may further include a vacuum pump (not illustrated) for providing suction force during suctional movement of the button member 60, and a vacuum pipe (not illustrated) connecting the vacuum pump and the button member suction hole 461. Meanwhile, the gripping method of the button member gripper 460 is not necessarily limited to the vacuum suction method, and the button member gripper 460 may include cases of transferring the button member 60 to the sensor device assembling part 200 by various methods such as a tongs-type method or a lifting method.

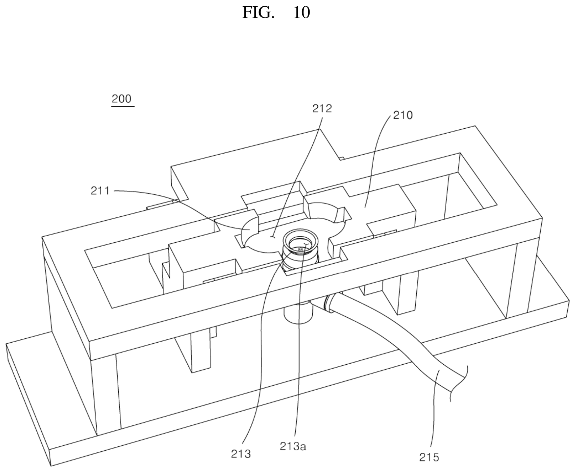

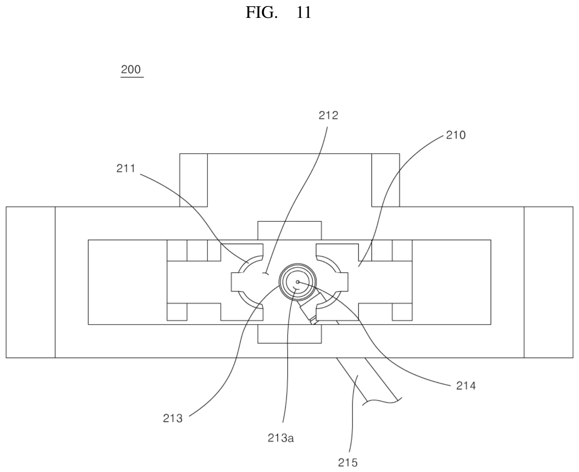

As illustrated in FIGS. 10 and 11, a sensor device assembling part 200 may include a sensor device assembly table 210, on which an intermediate assembly 1' is seated and assembly of a sensor device 1 is performed. The sensor device assembling part 200 may be provided with a sensor device seating groove 212 formed in a shape corresponding to a housing 10, and in a state where the intermediate assembly 1' is disposed in the sensor device seating groove 212, assembly of the sensor device 1 may be performed. On an inner surface of the sensor device assembly table 210 forming the sensor device seating groove 212, a sensor device seating surface 211 may be provided, which is inclined so that a lower end thereof is protruded toward the inside of the sensor device seating groove 212 more than an upper end thereof, and the sensor device seating surface 211 may have a rounded shape corresponding to an external shape of the housing 10. Since a lower side end of the intermediate assembly 1' disposed in the sensor device seating groove 212 is supported by the sensor device seating surface 211, the intermediate assembly 1' may be more firmly disposed in the sensor device seating groove 212.

At a lower portion of the sensor device seating groove 212, a sensor device support part 213 for supporting the intermediate assembly 1' during an assembly process of the sensor device 1 may be provided. An interior of the sensor device support part 213 may be provided with a support part internal space 213a having a shape with an open upper portion. At a lower side of the support part internal space 213a, a support suction hole 214 configured to suck air from the support part internal space 213a may be provided, and the sensor device support part 213 may be provided with a suction hose 215 connecting a vacuum pump (not illustrated) and the support suction hole 214. During assembly of the sensor device 1, a tip end portion of a needle body 91 coupled to an intermediate assembly 1' may be disposed inside the support part internal space 213a. During a process of inserting the sensor member 20 into the intermediate assembly 1' coupled with the needle 90, since the support suction hole 214 maintains a state of sucking air, a tip end of a sensor probe part 22 may sequentially pass through the needle holder incision part 94 and the needle body incision part 92 while maintaining alignment by the Bernoulli principle. The support suction hole 214 may be formed at a position corresponding to a tip end of a needle body 91 of the intermediate assembly 1' disposed in the sensor device seating groove 212, and the tip end portion of the needle body 91 may be disposed inside the support suction hole 214 during an assembly process of the sensor device 1.

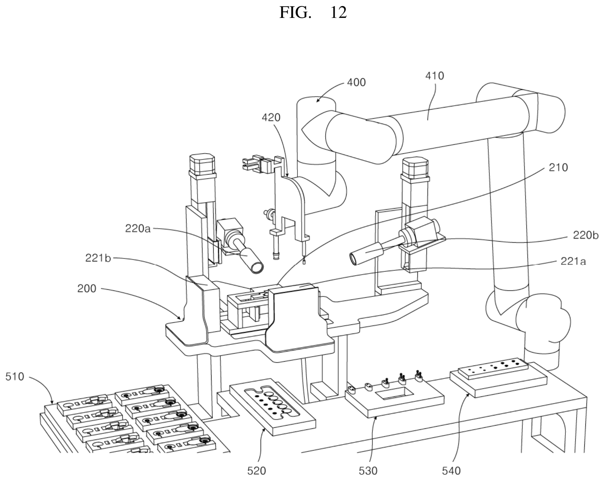

As illustrated in FIG. 12, the sensor device assembling part 200 may include a position sensor disposed at one side of the sensor device assembly table 210. The position sensor may detect whether the tip end portion of the needle body 91 is positioned at a central portion of the first housing through-hole 13 during insertion of the needle 90, or whether a tip end of the sensor probe part 22 is positioned at a central portion of the needle holder incision part 94 during insertion of the sensor member 20, and may transmit relevant signals related to these positions to a control part (not illustrated). When the control part (not illustrated) determines that the tip end portion of the needle body 91 deviates from the central portion of the first housing through-hole 13, or that the tip end of the sensor probe part 22 deviates from the central portion of the needle holder incision part 94, the control part may control to adjust positions of a first robot arm hand part 320 or a second robot arm hand part 420 so that the tip end portion of the needle body 91 or the tip end of the sensor probe part 22 is positioned respectively at the central portion of the first housing through-hole 13 or the central portion of the needle holder incision part 94.

The position sensor is not particularly limited as long as it is a means capable of measuring positions of the tip end portion of the needle body 91 or the tip end portion of the sensor probe part 22, but in terms of measurement accuracy, a vision sensor capable of measuring the positions of the tip end portion of the needle body 91 or the tip end portion of the sensor probe part 22 through captured images may be more preferable.

As a non-limiting example, the vision sensor may include a first vision camera 220a configured to capture a first housing through-hole 13 region or a needle holder incision part 94 region of an intermediate assembly 1' seated in a sensor device seating groove 212 from one side of the sensor device assembly table 210, and a second vision camera 220b configured to capture the first housing through-hole 13 region or the needle holder incision part 94 region of the intermediate assembly 1' seated in the sensor device seating groove 212 from a direction intersecting with a direction in which a lens of the first vision camera 220a is oriented. Since the first vision camera 220a and the second vision camera 220b are disposed to capture the first housing through-hole 13 region or the needle holder incision part 94 region from intersecting directions, whether the tip end portion of the needle body 91 or the tip end portion of the sensor probe part 22 is positioned at the central portion of the first housing through-hole 13 or the central portion of the needle holder incision part 94 can be measured in three dimensions. The vision sensor may further include a first lighting part 221a and a second lighting part 221b configured to irradiate light from positions facing the first vision camera 220a and the second vision camera 220b, respectively, to assist the imaging by the first vision camera 220a and the second vision camera 220b.

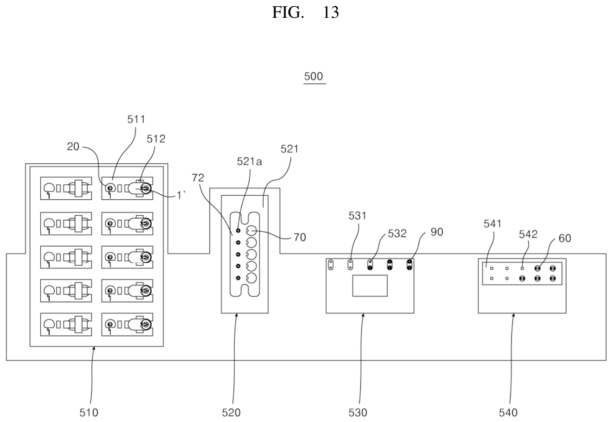

As illustrated in FIG. 13, a component supplying part 500 may include an intermediate assembly supplying table 510 configured to supply a sensor member 20 and an intermediate assembly 1' during assembly of a sensor device 1, a button cover supplying table 520 configured to supply a button cover 70, a needle supplying table 530 configured to supply a needle 90, and a button member supplying table 540 configured to supply a button member 60.

The intermediate assembly supplying table 510 may be provided with a plurality of intermediate assembly trays 511 in which the intermediate assembly 1' and the sensor member 20 are provided in pairs. During manufacturing of the intermediate assembly 1', firmware for communication connection with a specific sensor member 20 may be installed in the intermediate assembly 1'. In the intermediate assembly tray 511, the intermediate assembly 1' and the sensor member 20, which are intended to be assembled together, may be disposed in pairs respectively, and the sensor device manufacturing apparatus 100 may preferably sequentially move the intermediate assembly 1' and the sensor member 20 provided in one intermediate assembly tray 511 to the sensor device assembling part 200 to assemble the sensor device 1. The intermediate assembly tray 511 may be provided with grooves (reference numeral not assigned) disposed to respectively accommodate the intermediate assembly 1' and the sensor member 20 to prevent arbitrary movement thereof, and in a groove (reference numeral not assigned) on which the intermediate assembly 1' is seated, a housing gripper tongs introducing groove 512, into which a tip end portion of housing gripper tongs 341 may be introduced and disposed during a process of gripping the intermediate assembly 1' by the housing gripper tongs 341, may be additionally provided.

The button cover supplying table 520 may be provided with a button cover film tray 521 on which a film 72 having button covers 70 attached is disposed. The button cover film tray 521 may be provided with an auxiliary support 521a configured to assist in easily separating the button cover 70 from the film 72 during a process in which a button cover gripper 451 suction-separates the button cover 70 from the film 72. The auxiliary support 521a may be provided to support a lower side of the film 72 at a position corresponding to a central portion of each button cover 70, in a state where the film 72 with the button covers 70 attached is seated on the button cover film tray 521.

The needle supplying table 530 may be provided with a needle tray 531 on which the needle 90 is disposed. In the needle tray 531, a needle tray groove 532 having an open upper portion may be formed along a height direction, and the needle 90 may be disposed on the needle tray 531 by inserting at least a part of a needle body 91 into the needle tray groove 532.

The button member supplying table 540 may be provided with a button member tray 541 on which the button member 60 is disposed. In the button member tray 541, a button member tray groove 542 having an open upper portion may be formed along a height direction, and the button member 60 may be disposed on the button member tray 541 by inserting at least a part of the button member 60 into the button member tray groove 542.

From the viewpoints of production efficiency and product quantity management, as illustrated in FIG. 12, the sensor member 20, the intermediate assembly 1', the button cover 70, the needle 90, and the button member 60 may preferably be respectively provided on the intermediate assembly trays 511, the button cover film trays 521, the needle trays 531, and the button member trays 541 in corresponding quantities. Meanwhile, although the above description exemplarily describes the component supplying part 500 in which components are arranged on each tray, the configuration of the component supplying part 500 is not necessarily limited thereto, and the component supplying part 500 may include an automated component supplying part configured to continuously supply components to specific positions, so that end effectors corresponding to the respective components transfer the components to the sensor device assembling part 200.

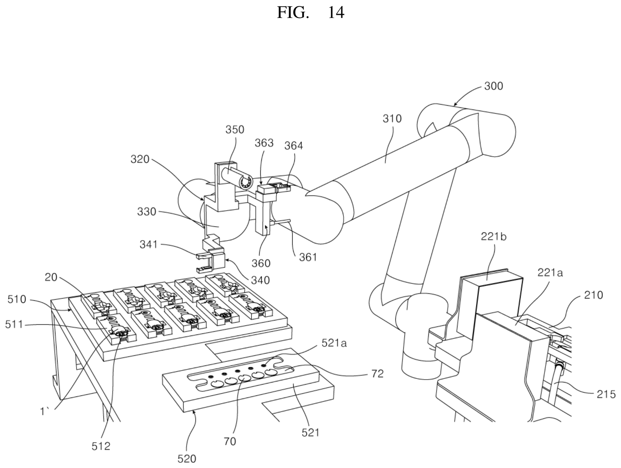

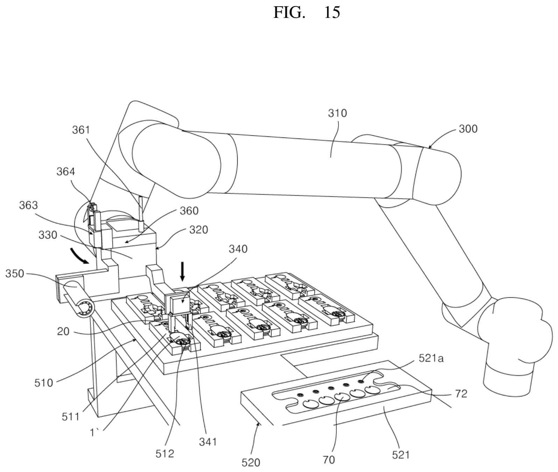

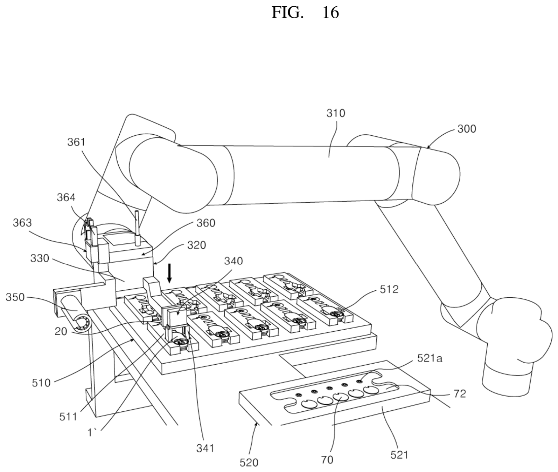

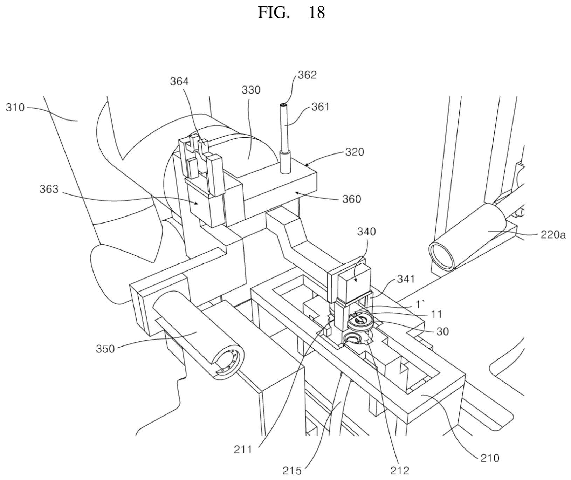

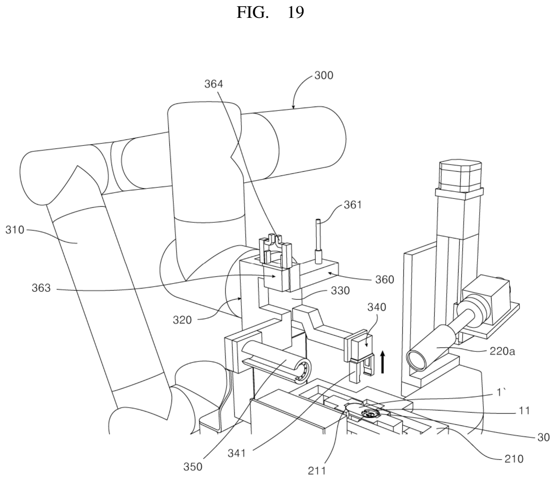

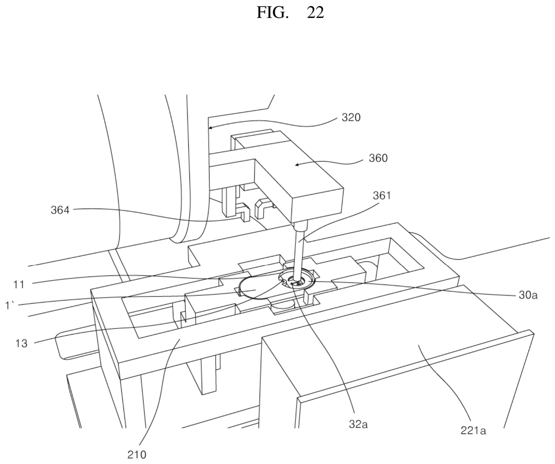

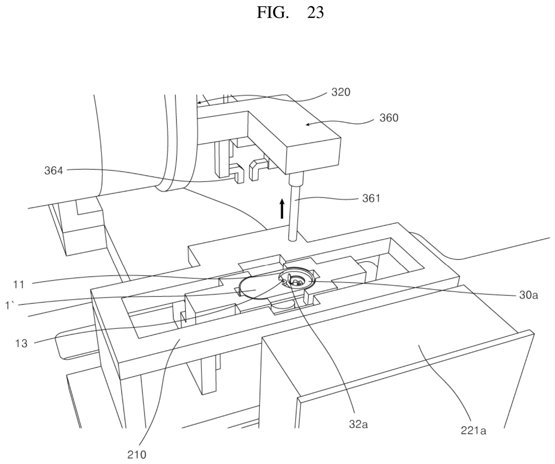

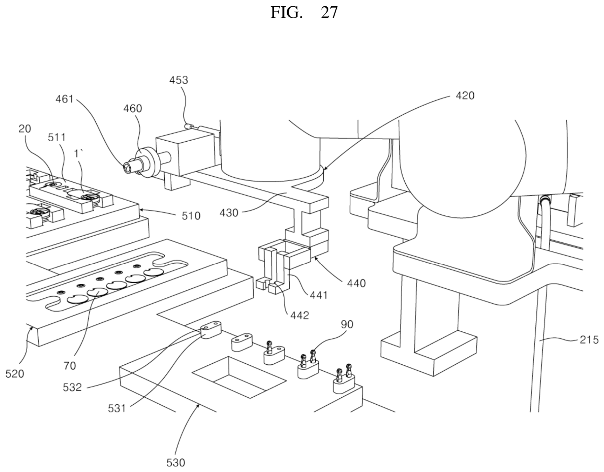

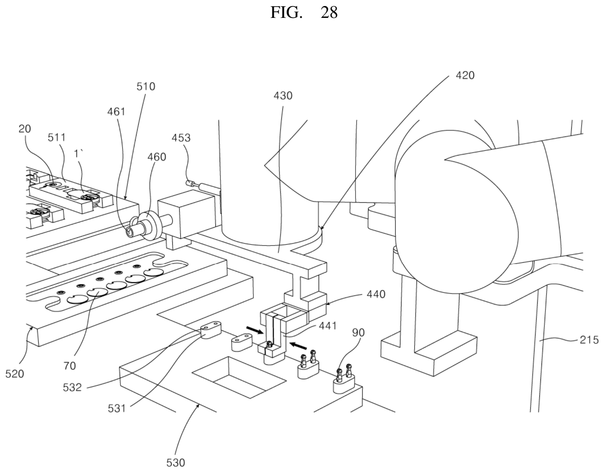

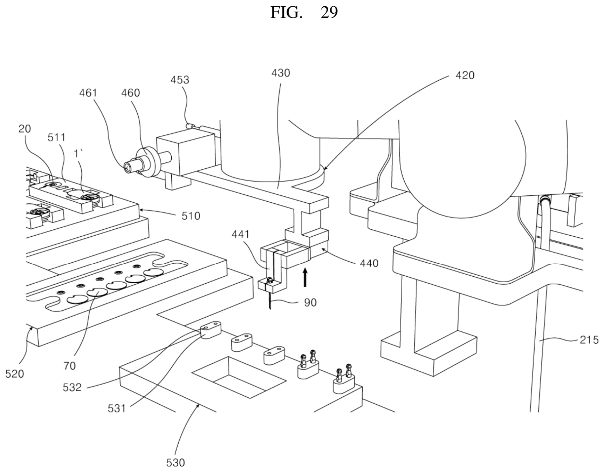

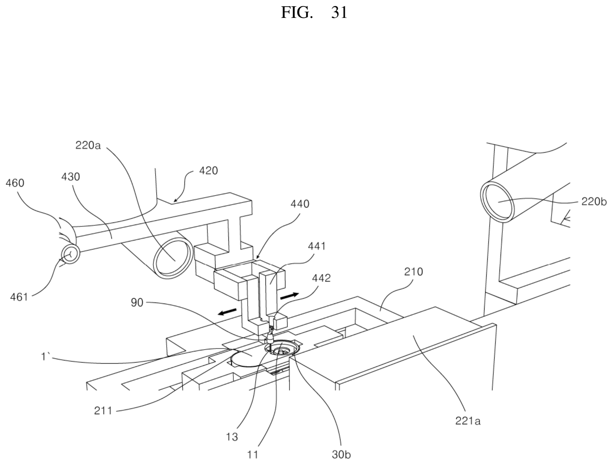

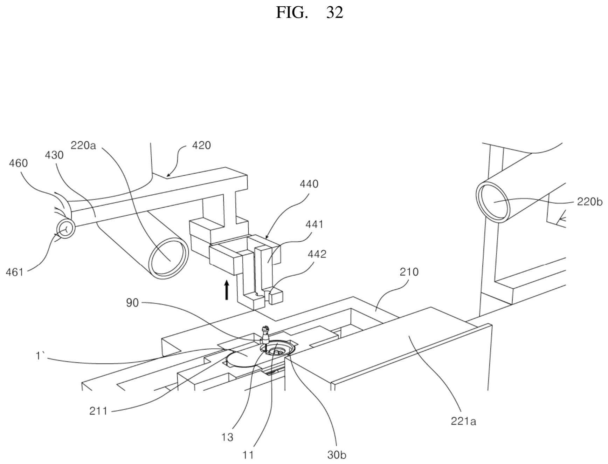

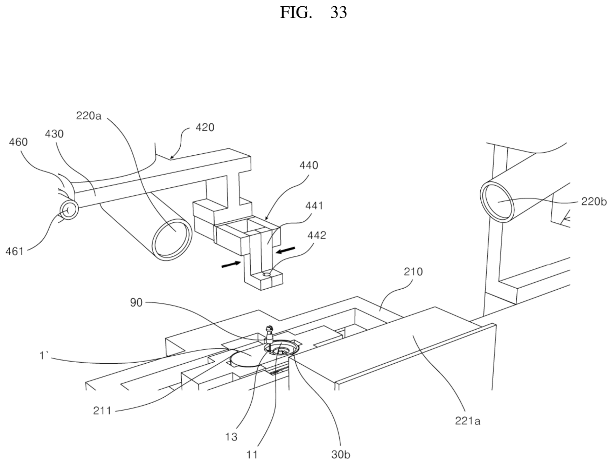

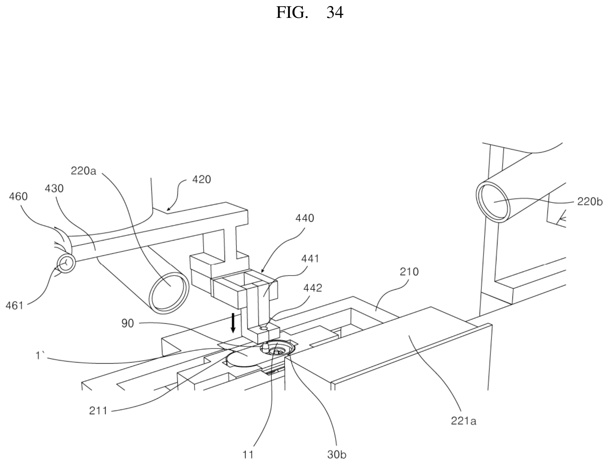

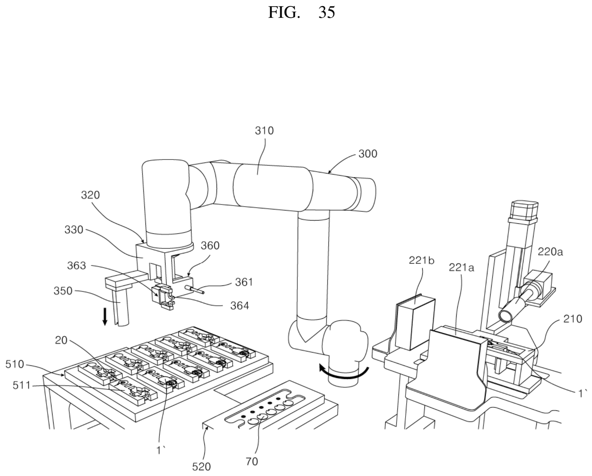

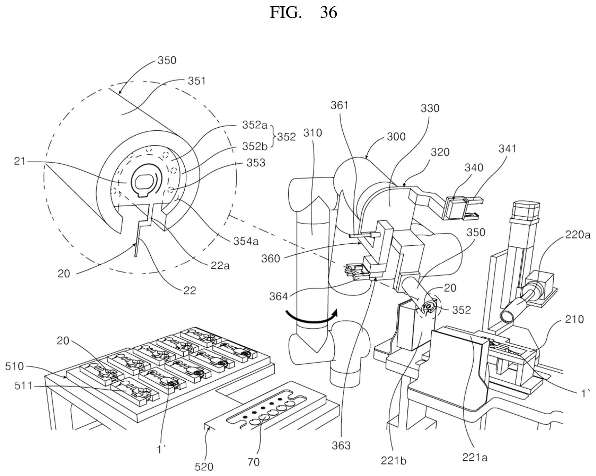

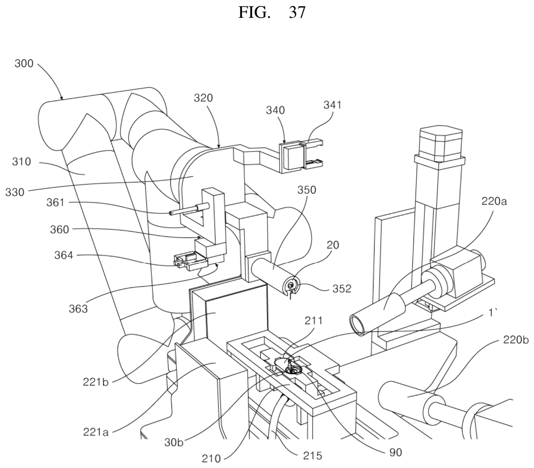

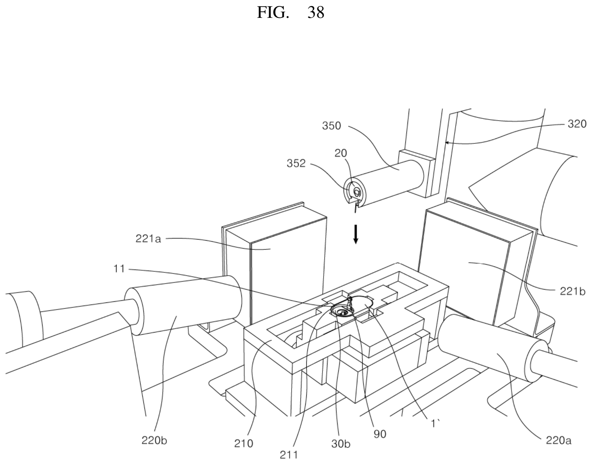

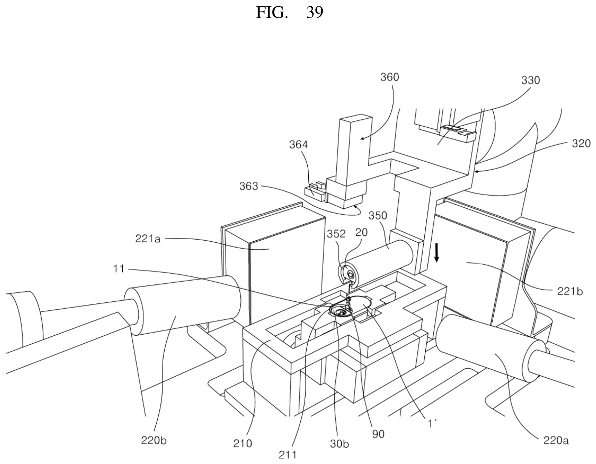

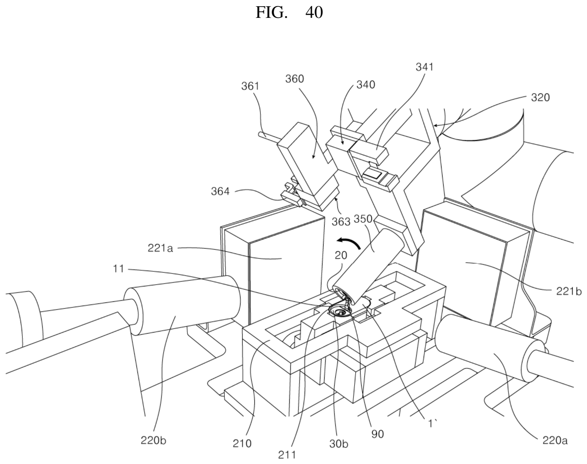

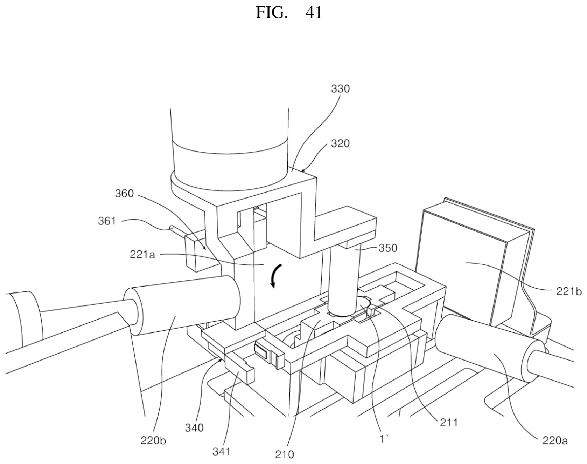

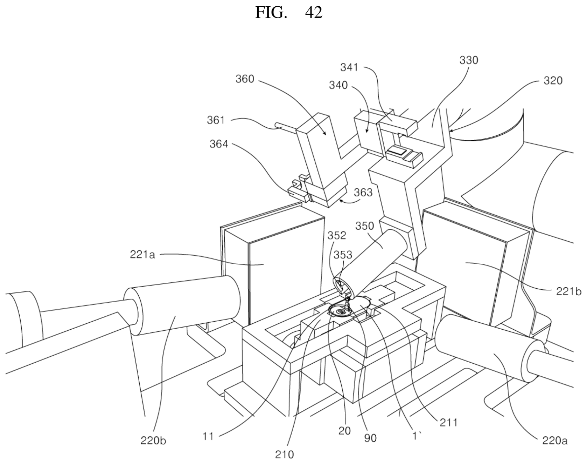

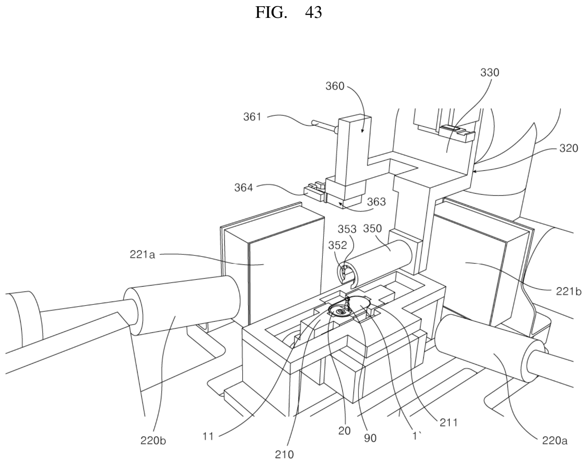

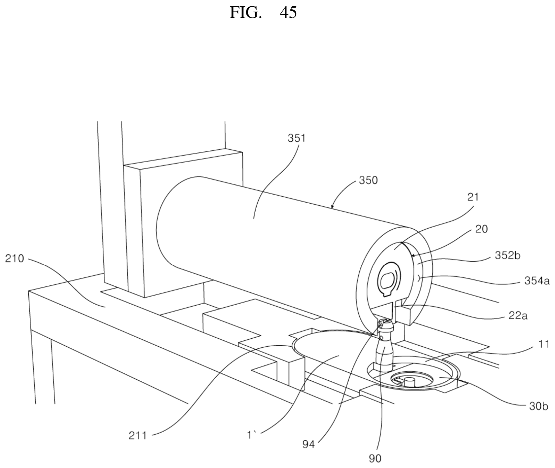

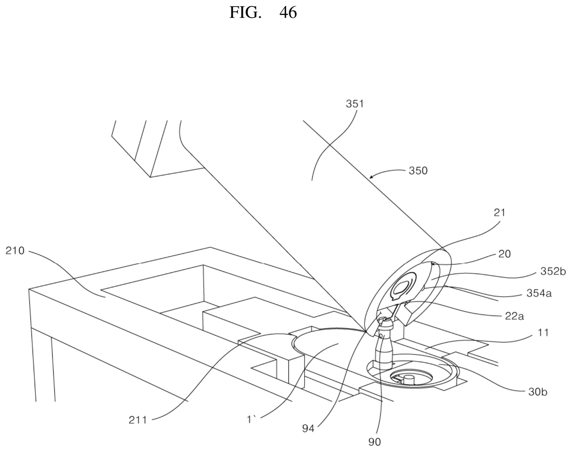

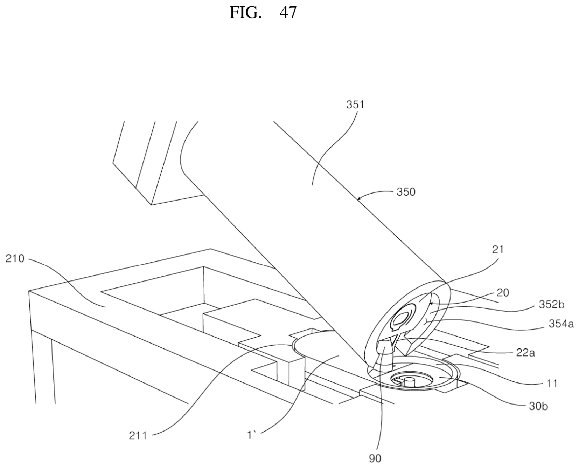

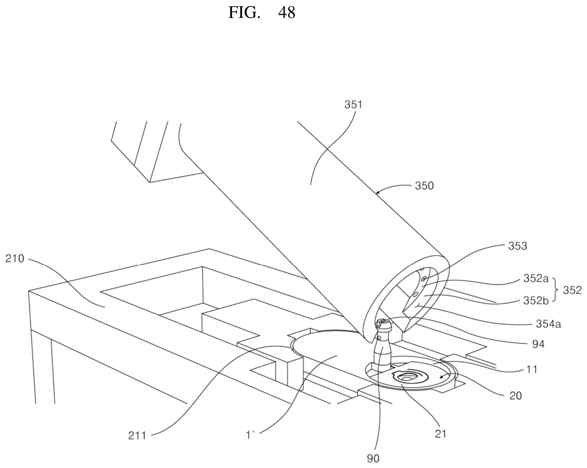

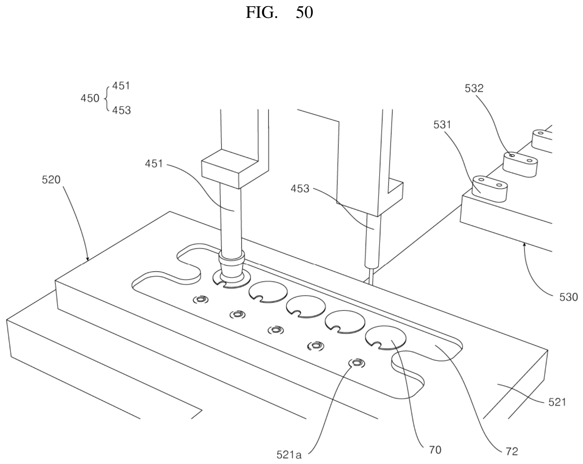

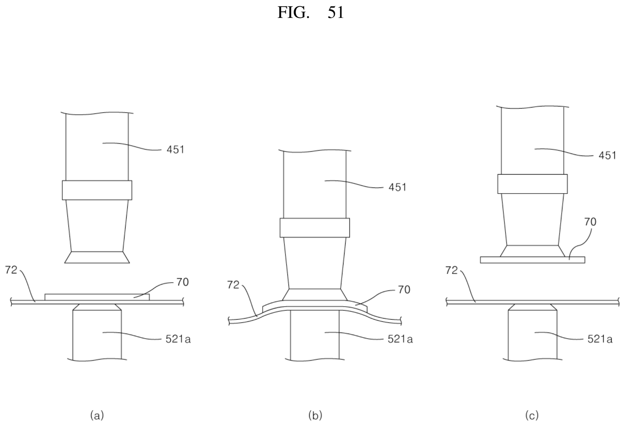

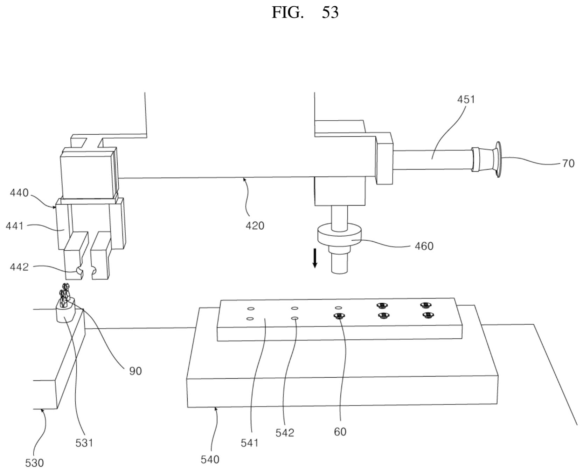

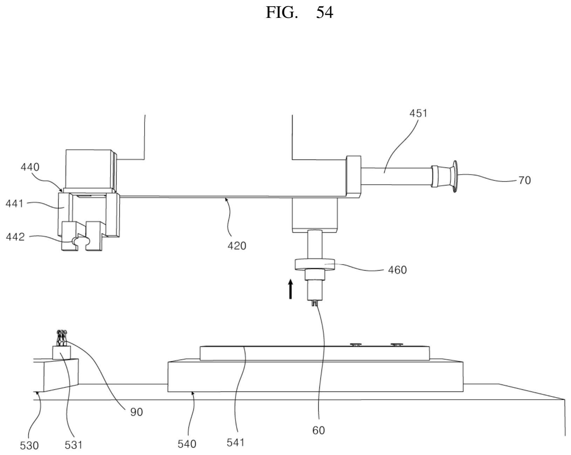

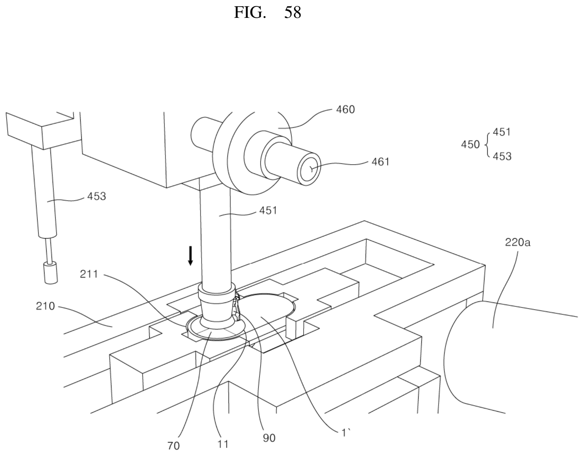

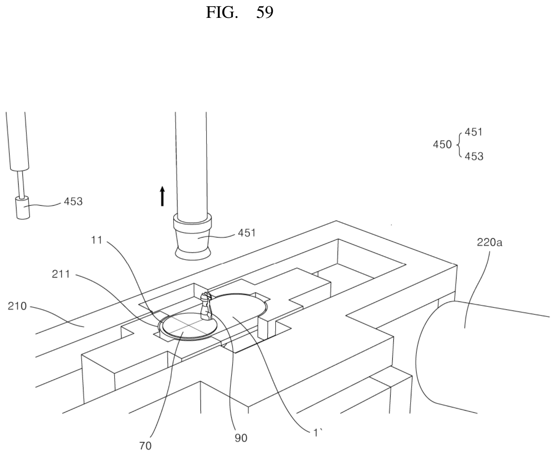

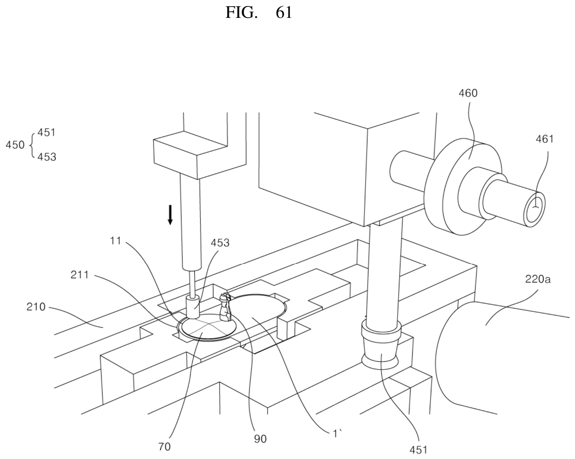

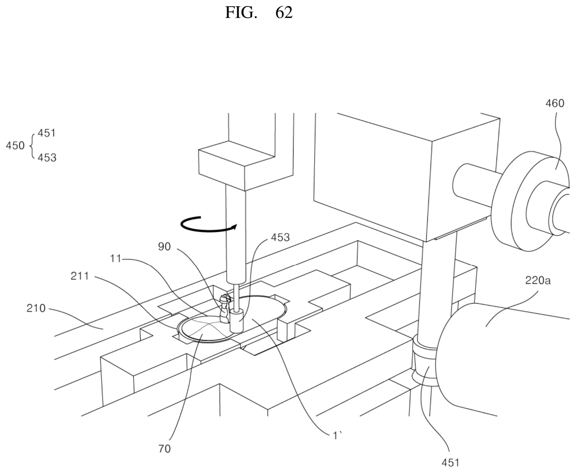

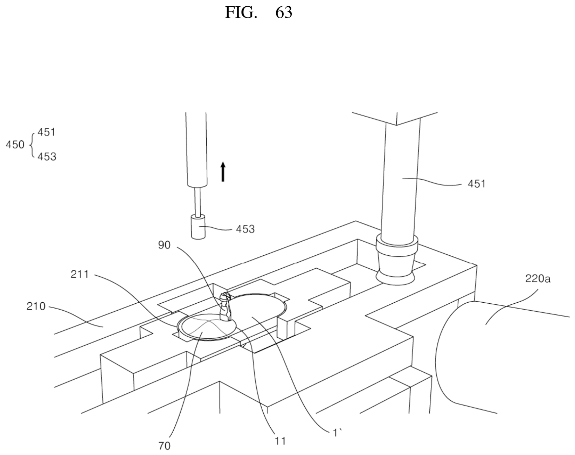

Hereinafter, with reference to FIGS. 14 to 63, a manufacturing process of a sensor device according to an aspect of the present invention by a manufacturing apparatus for a sensor device will be described in more detail.

Before manufacturing the sensor device 1 using the sensor device manufacturing apparatus 100 according to an aspect of the present invention, an operator may perform a preceding operation of disposing each component used for manufacturing the sensor device 1 on the component supplying part 500, and when using an automated component supplying part 500, the operation of disposing each component on the component supplying part 500 by the operator may be omitted.

As illustrated in FIGS. 14 to 16, after a first robot arm hand part 320 moves toward the intermediate assembly supplying table 510, a housing gripper 340 may descend toward an intermediate assembly 1' disposed on an intermediate assembly tray 511 and grip the intermediate assembly 1'. As the housing gripper 340 descends, a tip end portion of the housing gripper tongs 341 may be introduced into an interior of a housing gripper tongs introducing groove 512, and gripping of the intermediate assembly 1' may be performed in a state where the tip end portion of the housing gripper tongs 341 is disposed inside the housing gripper tongs introducing groove 512. At this time, the housing gripper tongs 341 may grip the intermediate assembly 1' so that both side ends of the intermediate assembly 1' closely contact housing seating parts 342 of the housing gripper tongs 341.

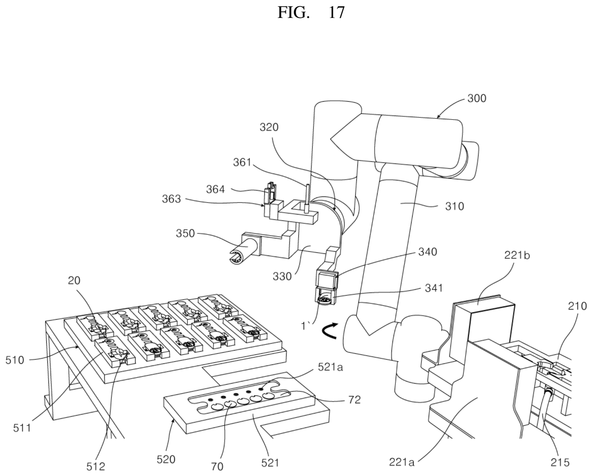

As illustrated in FIGS. 17 to 19, the first robot arm hand part 320 may move toward the sensor device assembling part 200 while maintaining a state in which the housing gripper 340 grips the intermediate assembly 1'. The housing gripper tongs 341, after transferring the intermediate assembly 1' to a sensor device seating groove 212, may move in a retracting direction from the sensor device assembly table 210.

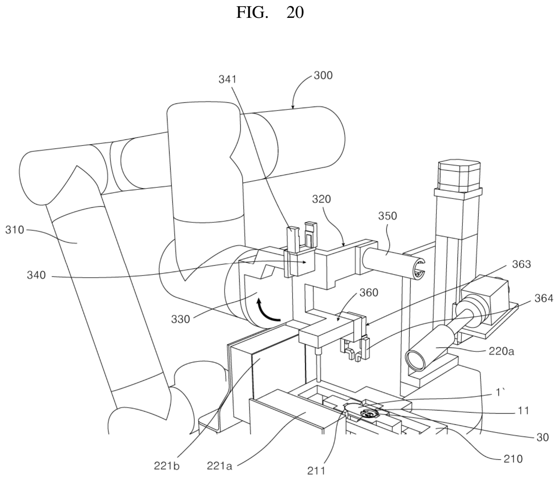

As illustrated in FIG. 20, after the housing gripper 340 transfers the intermediate assembly 1' onto the sensor device assembly table 210, a first rotation plate 330 may rotationally move, and as the first rotation plate 330 rotationally moves, a release paper removing unit 360 may switch to a state facing the intermediate assembly 1' seated on the sensor device assembly table 210.

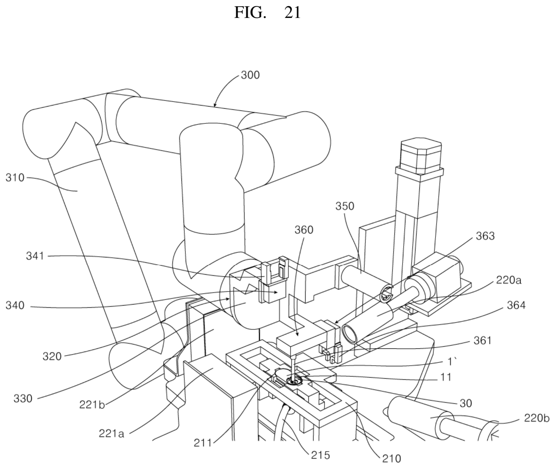

As illustrated in FIGS. 21 to 23, a tip end portion of a release paper suction rod 361 having a release paper suction hole 362 may move to a position where it contacts a tip end portion of a release paper gripping part 32a, and suction of air at the release paper suction hole 362 may be initiated so that the release paper gripping part 32a is vacuum-suctioned onto the tip end portion of the release paper suction rod 361. The point in time of initiating air suction at the release paper suction hole 362 is not particularly limited, but in terms of suction accuracy of the release paper gripping part 32a, it may be preferable that the air suction at the release paper suction hole 362 be initiated simultaneously with or immediately after the tip end portion of the release paper suction rod 361 contacts the release paper gripping part 32a. The release paper suction rod 361, while maintaining a state in which the release paper gripping part 32a is vacuum-suctioned to its tip end portion, may move upward, and as the release paper suction rod 361 moves upward, the tip end portion of the release paper gripping part 32a may move upward together with the release paper suction rod 361. That is, since the release paper suction rod 361 moves upward while vacuum-suctioning the tip end portion side of the release paper gripping part 32a, the release paper gripping part 32a may be deformed into a folded state so that the tip end portion side thereof is directed upward.

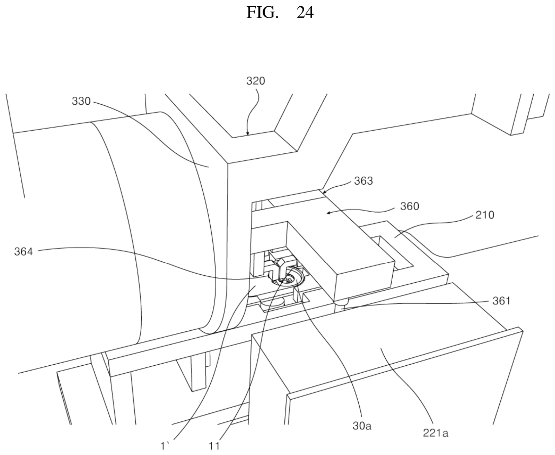

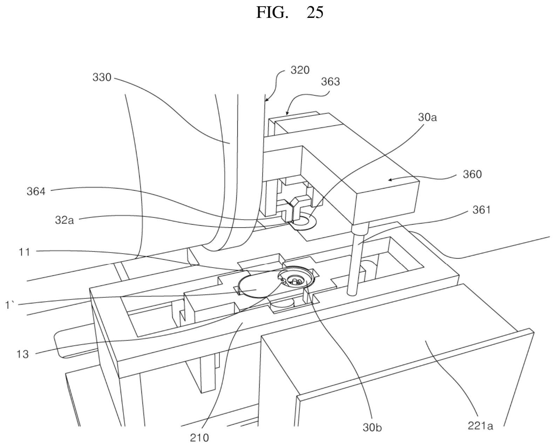

As illustrated in FIGS. 24 and 25, after the release paper gripping part 32a is deformed into a folded state, air suction at the release paper suction hole 362 may be terminated, and the release paper suction rod 361 may retract and move away from the sensor device assembly table 210. Subsequently, a release paper gripper 363 may move to a position capable of gripping the release paper gripping part 32a, and release paper gripper tongs 364 may grip the release paper gripping part 32a, the tip end portion of which is lifted. The release paper gripper tongs 364, while maintaining a state of gripping the release paper gripping part 32a, may move in a retracting direction from the sensor device assembly table 210, and accordingly, the release paper 30a may be removed from the intermediate assembly 1'. The sensor device manufacturing apparatus 100 according to an aspect of the present invention may further include a waste container (not illustrated) configured to collect waste generated during an assembly process of the sensor device, and the release paper gripper 363 gripping the release paper 30a may move toward the waste container (not illustrated) to discharge the release paper 30a, separated from the intermediate assembly 1', into the waste container (not illustrated). As the release paper gripper 363 removes the release paper 30a from the intermediate assembly 1', an adhesive sheet 30b disposed on a sensor member seating part 11 may be exposed to the outside.

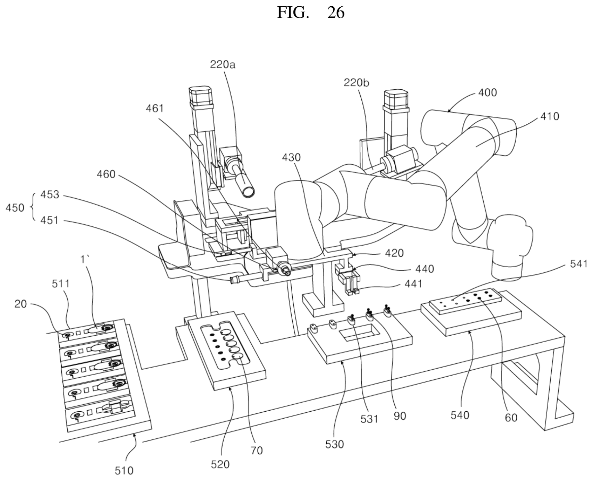

As illustrated in FIGS. 26 to 29, after the release paper gripper 363 removes the release paper 30a or during a process in which the release paper gripper 363 removes the release paper 30a by the release paper gripper 363, a second robot arm hand part 420 may move toward a needle supplying table 530. A second rotation plate 430 may rotationally move so that a needle gripper 440 faces the needle tray 531. After the needle gripper tongs 441 descend so that the needle seating part 442 are positioned at both sides of a needle holder 93 of a needle 90 disposed on the needle tray 531, the needle gripper tongs 441 may be closed, thereby gripping the needle 90. While the needle gripper tongs 441 maintain a state of gripping the needle 90, the needle holder 93 may be disposed in an interior of the needle seating part 442 or maintain a state of being closely contacted with the needle seating part 442. The needle gripper 440 may ascend while gripping the needle 90, and the needle 90 gripped by the needle gripper 440 may be separated from the needle tray 531 and move together with the needle gripper 440.

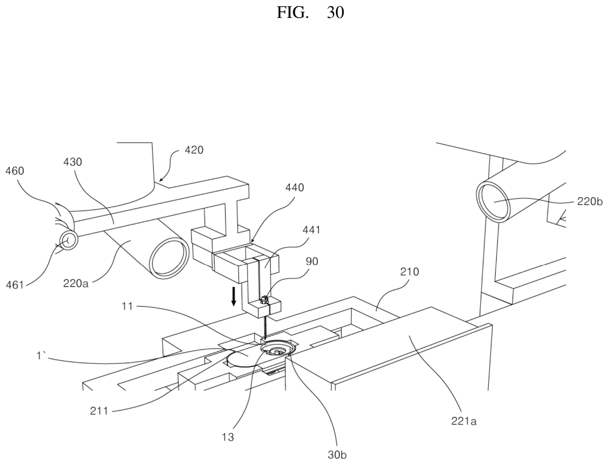

As illustrated in FIGS. 30 to 34, the needle gripper 440 gripping the needle 90 may move toward the sensor device assembly table 210 on which the intermediate assembly 1' is seated. The first vision camera 220a and the second vision camera 220b may capture a tip end portion region of the needle body 91 and a first housing through-hole 13 region when the needle 90 gripped by the needle gripper tongs 441 moves to an upper side of the intermediate assembly 1', and may transfer a first position signal to a control part (not illustrated), which may control to adjust a position of the second robot arm hand part 420 based on the first position signal by determining whether the tip end portion of the needle body 91 is positioned at a central portion of the first housing through-hole 13. Through images captured by the first vision camera 220a and the second vision camera 220b, when it is determined that the tip end portion of the needle body 91 is positioned at a position corresponding to the central portion of the first housing through-hole 13, the needle gripper 440 may move downward so that the tip end portion of the needle body 91 is inserted into the first housing through-hole 13. For more precise insertion of a needle body 91, the needle gripper tongs 441 may be switched to an opened state in which the needle gripper tongs 441 do not grip the needle 90, while the needle body 91 is not yet completely inserted into the first housing through-hole 13 and the second housing through-hole 14, and after moving upward in the state of not gripping the needle 90, the needle gripper tongs 441 may be switched to a closed state and then move downward again. Since the needle gripper tongs 441 move downward toward the needle 90 in a closed state, the upper end portion of the needle 90 may be pressed downward by being contacted with a lower end portion of the needle gripper tongs 441. As the needle gripper tongs 441 contact the upper end portion of the needle 90 and move downward, the needle body 91 may be completely inserted through the first housing through-hole 13 and the second housing through-hole 14.

As illustrated in FIGS. 26 to 48, after the needle body 91 is inserted into the first housing through-hole 13 and the second housing through-hole 14 of the intermediate assembly 1', or during the process of inserting the needle body 91 into the first housing through-hole 13 and the second housing through-hole 14 of the intermediate assembly 1', a first robot arm hand part 320 may move toward an intermediate assembly supplying table 510. A first rotation plate 330 may rotationally move so that a sensor member gripper 350 faces an intermediate assembly tray 511, and the sensor member gripper 350 may descend toward a sensor member 20 disposed on the intermediate assembly tray 511. The sensor member gripper 350 may descend toward the intermediate assembly tray 511 on which the intermediate assembly 1', currently being assembled on the sensor device assembly table 210, had been disposed, and may grip a sensor member 20 corresponding in software to the intermediate assembly 1' currently being assembled on the sensor device assembly table 210.

The sensor member gripper 350 may move to a position at which the sensor member gripping part 352 formed at one end of the sensor member gripper body 351 faces the sensor body part 21 of the sensor member 20 disposed on the intermediate assembly tray 511.. At a position where the sensor member gripping part 352 becomes adjacent to the sensor body part 21, or at a position where the sensor member gripping part 352 is in close contact with the sensor body part 21, air suction at the sensor member suction holes 353 may be initiated. As the air suction at the sensor member suction holes 353 is initiated, the sensor body part 21 may maintain a state of being vacuum-suctioned to the sensor member gripping part 352.

The sensor member gripper 350 may move toward the sensor device assembly table 210 while maintaining a state of vacuum-suctioning the sensor member 20. The sensor member gripper body 351 may be disposed such that a tip end portion of a sensor probe part 22 faces downward so that the sensor probe part 22 may be introduced into a needle body 91 through a needle holder incision part 94. That is, the sensor member gripper body 351 may be disposed such that a sensor member gripper incision part 355 faces a lower side, and accordingly, a sensor probe part 22 of a sensor member 20, vacuum-suctioned to a sensor member gripping part 352, may be exposed to an exterior of a lower side of the sensor member gripper body 351 through the sensor member gripper incision part 355.

The first vision camera 220a and the second vision camera 220b may capture a sensor probe part 22 tip end portion region and a needle holder incision part 94 region when the sensor member gripper body 351, to which the sensor member 20 is vacuum-suctioned, moves to an upper side of the intermediate assembly 1', and may transfer a second position signal to a control part (not illustrated). The control part (not illustrated) may control to adjust a position of the first robot arm hand part 320 by determining whether a tip end portion of the sensor probe part 22 is positioned at a central portion of the needle holder incision part 94, based on the second position signal. When it is determined, through images captured by the first vision camera 220a and the second vision camera 220b, that the tip end portion of the sensor probe part 22 is positioned at a position corresponding to the central portion of the needle holder incision part 94, the sensor member gripper 350 may descend and insert and dispose the sensor probe part 22 into the interior of the needle body 91 through the needle holder incision part 94. During a process in which the sensor probe part 22 is introduced through the needle holder incision part 94, air suction may be performed at a support suction hole 214. The point in time of initiating air suction at the support suction hole 214 is not particularly limited, but for alignment of the sensor probe part 22, it may be preferable that air suction at the support suction hole 214 be initiated before or simultaneously with introduction of the tip end portion of the sensor probe part 22 into the needle holder incision part 94. Meanwhile, the point in time of terminating air suction at the support suction hole 214 is also not particularly limited, but as described later, in order to assist adhesion of the sensor body part 21 to an adhesive sheet 30b disposed on the sensor member seating part 11, it may be more preferable that air suction at the support suction hole 214 be terminated after the sensor body part 21 is attached to the adhesive sheet 30b.

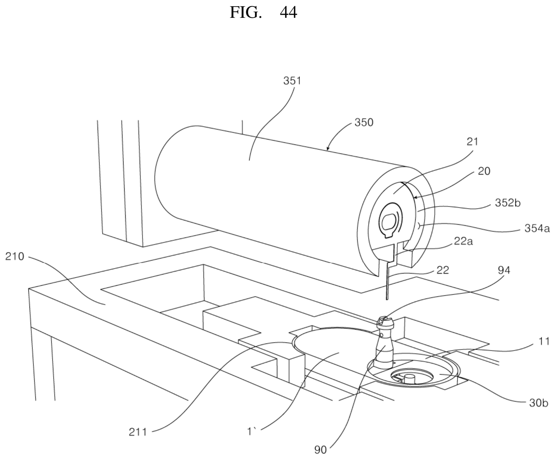

Hereinafter, with reference to FIGS. 44 to 48, behavior of the sensor member gripper 350 during an insertion process of the sensor member 20 will be described in more detail. The sensor member gripper body 351, to which the sensor member 20 is vacuum-suctioned, may be disposed parallel to a direction substantially perpendicular to the needle 90 at an upper side of the intermediate assembly 1', to which the needle 90 is coupled, and a tip end portion of the sensor probe part 22, exposed to an outer lower side of the sensor member gripper body 351 through the sensor member gripper incision part 355, may be disposed at an upper side of the needle holder incision part 94. The first vision camera 220a and the second vision camera 220b may capture a sensor probe part 22 tip end portion region and a needle holder incision part 94 region, and may transfer a second position signal to a control part (not illustrated), and the control part (not illustrated) may control to adjust a position of the first robot arm hand part 320 by determining whether the tip end portion of the sensor probe part 22 is positioned at a central portion of the needle holder incision part 94, based on the second position signal. When it is determined that the tip end portion of the sensor probe part 22 is positioned at the central portion of the needle holder incision part 94, the sensor member gripper 350 may descend, and as the sensor member gripper 350, to which the sensor member 20 is vacuum-suctioned, descends, the sensor probe part 22 may be introduced into the needle body 91 through the needle holder incision part 94.

When an extension part 22a is introduced into an interior of the needle holder incision part 94, the sensor member gripper 350 may stop descending, and the sensor member gripper body 351 may rotationally move to be disposed substantially parallel to the needle 90. As the sensor member gripper body 351 rotationally moves, an extension part 22a may be bent, and the sensor body part 21 may be disposed substantially parallel to the sensor member seating part 11. The needle holder 93 may be disposed in the sensor member gripper incision part 355 during a rotational movement process of the sensor member gripper body 351, and since the needle holder 93 is disposed in the sensor member gripper incision part 355 so that direct contact with the sensor member gripper body 351 does not occur, rotation movement interference of the sensor member gripper body 351 by the needle holder 93 may be effectively eliminated. During the rotation movement of the gripper body 351 or after the rotation movement of the gripper body 351 is terminated, the sensor member gripper body 351 may further descend, and after the sensor body part 21 moves to a position corresponding to the sensor member seating part 11, air suction at the sensor member suction holes 353 may be terminated. As air suction at the sensor member suction holes 353 is terminated, vacuum suction of the sensor member 20 by the sensor member gripper 350 may be ended, and the sensor body part 21 may be transferred and seated on the sensor member seating part 11. During a transferring process of the sensor body part 21, an air suction state at the support suction hole 214 may be maintained, and as air is suctioned through the support suction hole 214, the sensor body part 21 may be more firmly attached to an adhesive sheet 30b disposed on the sensor member seating part 11. After the sensor body part 21 is seated on the sensor member seating part 11, the sensor member gripper 350 may move upward while retracting, and to avoid interference with the needle holder 93 during the retraction movement of the sensor member gripper 350, the sensor member gripper body 351 may rotate in a direction opposite to the rotation direction during descent and move upward while retracting.

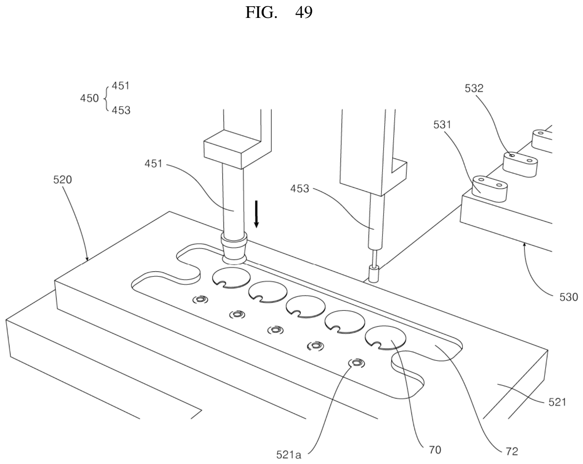

As illustrated in FIGS. 49 to 51, a second robot arm hand part 420 may sequentially pass through a button cover supplying table 520 and a button member supplying table 540 after or during seating of the sensor member on the sensor member seating part 11 of the intermediate assembly 1'.

A button cover gripper 451 may descend toward a button cover 70 disposed on a button cover film tray 521 from an upper side of the button cover supplying table 520. The button cover gripper 451 may descend so that one end of the button cover gripper 451, in which a button cover suction hole 452 is formed, comes into close contact with a central portion of an upper surface of the button cover 70, and as air is suctioned through the button cover suction hole 452, the button cover 70 may be vacuum-suctioned to the button cover gripper 451. Since a ring-shaped adhesive surface is provided along a circumferential direction of the button cover 70 on a lower surface of the button cover 70, which contacts a film 72, even under vacuum suction through the button cover suction hole 452, cases may occur in which the button cover 70 is not normally separated from the film 72. The auxiliary support 521a may be provided to support a lower portion of the film 72 at a position corresponding to a central portion of each button cover 70, and thus, while the lower portion of the film 72 is supported by the auxiliary support 521a, vacuum suction of the button cover 70 by the button cover gripper 451 may be initiated, and during upward movement of the button cover gripper 451, the button cover 70 may be more easily separated from the film 72. The auxiliary support 521a may further include an elastic member (not illustrated) that presses the film 72 upward to absorb impact during downward movement of the button cover gripper 451 and to allow the button cover 70 to be more easily separated.

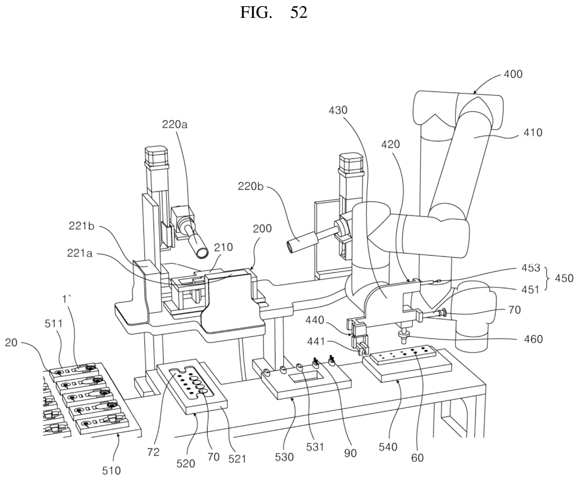

As illustrated in FIGS. 52 to 54, a button member gripper 460 may move to an upper side of a button member supplying table 540 while a button cover gripper 451 is in a state of vacuum-suctioning a button cover 70, and then may descend toward a button member 60 disposed on the button member supplying table 540. The button member gripper 460 may descend toward the button member 60 while a button member suction hole 461 formed at one end of the button member gripper 460 faces downward. The button member suction hole 461 may suction air in a state in which one end of the button member 60 is in close contact with the button member suction hole 461, and accordingly, the button member 60 may be vacuum-suctioned to the button member gripper 460. The button member gripper 460 may move upward together with the button member 60 while maintaining a state of vacuum-suctioning the button member 60, and the second robot arm hand part 420 may move toward a sensor device assembly table 210 while the button cover 70 and the button member 60 are vacuum-suctioned to the button cover gripper 451 and the button member gripper 460, respectively.

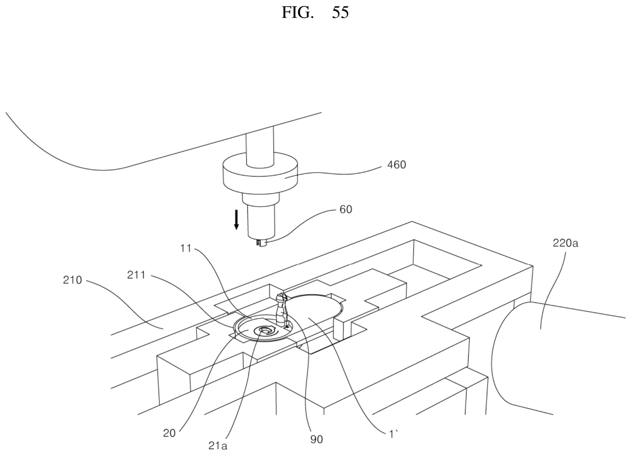

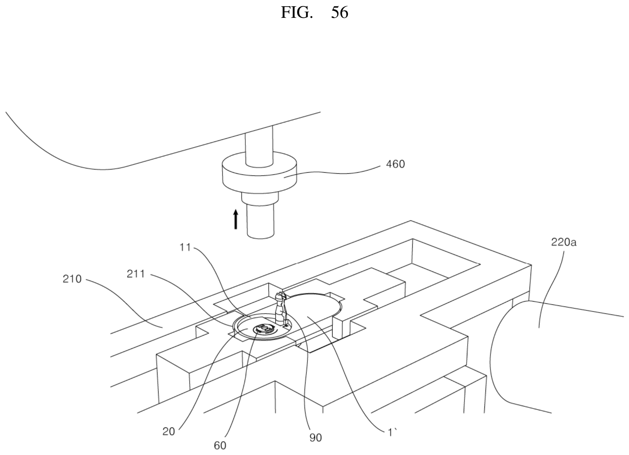

As illustrated in FIGS. 55 and 56, the button member gripper 460, to which the button member 60 is vacuum-suctioned, may move to an upper side of an intermediate assembly 1' disposed on the sensor device assembly table 210. The button member gripper 460 may move such that the button member 60, vacuum-suctioned to one end of the button member gripper 460, is positioned at a position corresponding to the sensor body part through-hole 21a of the intermediate assembly 1', and then may descend. During insertion of the button member 60, position correction based on a position sensor may be performed. The button member gripper 460 may terminate vacuum suction after descending to a position where the button member 60, vacuum-suctioned to the button member gripper 460, is inserted into the sensor body part through-hole 21a. After terminating vacuum suction, the button member gripper 460 may move upward while retracting, and the button member 60 may remain in a state inserted into the sensor body part through-hole 21a.

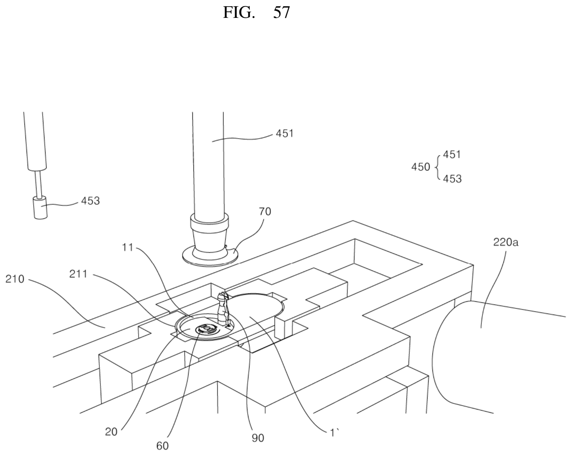

As illustrated in FIGS. 57 to 59, the button cover gripper 451 may move to an upper side of the intermediate assembly 1' after the button member 60 is disposed in the sensor body part through-hole 21a. The button cover gripper 451 may move such that the button cover 70, vacuum-suctioned to one end of the button cover gripper 451, is positioned at a position corresponding to a sensor member seating part 11 of the intermediate assembly 1', and then may descend. The button cover gripper 451 may descend to a position where a button cover 70, vacuum-suctioned to the button cover gripper 451, comes into close contact with a sensor member seating part 11, and then may terminate vacuum suction. The button cover 70 may be attached to the sensor member seating part 11 by means of an adhesive surface provided on one surface of the button cover 70. After termination of vacuum suction, the button cover gripper 451 may move upward, and the button cover 70 may remain in a state attached to the sensor member seating part 11.

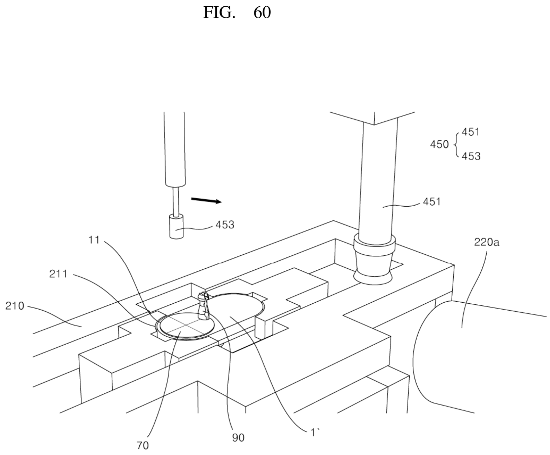

As illustrated in FIGS. 60 to 63, a button cover presser 453 may move to an upper side of the button cover 70 after the button cover 70 is disposed on the sensor member seating part 11. The button cover presser 453 may descend to a position where a tip end portion of the button cover presser 453 presses an edge portion of the button cover 70, and may move along a circumferential direction of the button cover 70 to press the edge portion of the button cover 70 downward. As the button cover presser 453 presses the edge portion of the button cover 70, the adhesive surface provided on the button cover 70 may be more firmly attached to the sensor member seating part 11.

The second robot arm hand part 420 may move to an upper side of the sensor device assembly table 210 after attachment of the button cover 70 is completed, and the housing gripper 340 may grip an assembled sensor device 1 and discharge the assembled sensor device 1' from the sensor device assembly table 210.

As described above, the sensor device manufacturing apparatus 100 according to one aspect of the present invention transports components constituting a sensor device 1 using a vacuum suction method to assemble the sensor device 1, thereby effectively preventing product defects occurring during manufacturing of the sensor device 1. Furthermore, since the sensor device 1 is manufactured by an automated facility, workability, productivity, and economic feasibility may be effectively improved.

Although the present invention has been described in detail through the embodiments above, other forms of embodiments are also possible. Therefore, the technical teachings and scope of the claims described below are not limited to the embodiments.

**[Description of Reference Numerals]**

| | | | |
|---|---|---|---|
| 1: | Sensor device | 1': | Intermediate assembly |
| 10: | Housing | 20: | Sensor member |
| 30: | Sensor adhesive member | 40: | Electronic board |
| 50: | Battery | 60: | Button member |
| 70: | Button cover | 80: | Housing adhesive member |
| 100: | Sensor device manufacturing appar atus | | |
| 200: | Sensor device assembling part | 300: | First robot arm |
| 310: | First robot arm joint part | 320: | First robot arm hand part |
| 330: | First rotation plate | 340: | Housing gripper |
| 350: | Sensor member gripper | 360: | Release paper removing unit |
| 361: | Release paper suction rod | 363: | Release paper gripper |
| 400: | Second robot arm | 410: | Second robot arm joint part |
| 420: | Second robot arm hand part | 430: | Second rotation plate |
| 440: | Needle gripper | 450: | Button cover attaching unit |
| 451: | Button cover gripper | 453: | Button cover presser |
| 460: | Button member gripper | 500: | Component supplying part |
| 510: | Intermediate assembly supplying table | | |
| 520: | Button cover supplying table | 530: | Needle supplying table |
| 540: | Button member supplying table | | |

## Claims

1. A manufacturing apparatus for a sensor device, comprising:
a component supplying part in which components of a sensor device are disposed;
a sensor device assembling part in which the sensor device is assembled by receiving the component transferred from the component supplying part;
an operating part including a terminal end portion having a suction hole formed thereon for vacuum suction, the terminal end portion configured to transfer at least one of the components to the sensor device assembling part by vacuum suction; and
a control part configured to control an operation of the operating part.

2. The manufacturing apparatus of claim 1, wherein the sensor device assembling part comprises:
a sensor device assembly table on which the sensor device in an assembly stage is disposed; and
a position sensor disposed at one side of the sensor device assembly table and configured to detect position information of at least one of the components transferred to the sensor device assembly table, and
wherein the control part controls the operating part to adjust a position of at least one terminal end portion based on the position information.

3. The manufacturing apparatus of claim 2, wherein the position sensor comprises a camera capable of image capturing, and
wherein the control part controls the operating part based on image information captured by the camera.

4. The manufacturing apparatus of claim 1, wherein the component comprises:
an intermediate assembly including a housing forming an external shape of the sensor device; and
a plate-shaped sensor member disposed on the housing such that a tip end thereof is inserted into a transcutaneous position of a human body to detect biological information.

5. The manufacturing apparatus of claim 4, wherein the terminal end portion comprises a sensor member gripper configured to grip the sensor member by vacuum suction and move the sensor member to the sensor device assembling part, and
wherein the sensor member gripper transfers the sensor member to the intermediate assembly disposed in the sensor device assembling part.

6. The manufacturing apparatus of claim 5, wherein the sensor member gripper comprises a sensor member gripper body including, at a tip end portion thereof, a sensor member gripping part recessedly formed in a shape corresponding to at least a part of the sensor member, and
wherein one surface of the gripper body forming the sensor member gripping part is provided with a plurality of sensor member suction holes provided to suction external air.

7. The manufacturing apparatus of claim 5, wherein the sensor member comprises:
a plate-shaped sensor body part disposed on the housing; and
a sensor probe part extending from one end of the sensor body part and having a tip end portion disposed outside the housing, and
wherein the sensor member gripper rotationally moves during a process of inserting the sensor member into the intermediate assembly such that the sensor body part and the sensor probe part are disposed on different planes.

8. The manufacturing apparatus of claim 4, wherein the terminal end portion further comprises a housing gripper capable of transferring the intermediate assembly to the sensor device assembling part while moving in a state of gripping the intermediate assembly.

9. The manufacturing apparatus of claim 4, wherein the component supplying part comprises an intermediate assembly tray in which the intermediate assembly and the sensor member are disposed in pairs, and
wherein the terminal end portion transfers the intermediate assembly and the sensor member disposed in one intermediate assembly tray to the sensor device assembling part to assemble the sensor device.

10. The manufacturing apparatus of claim 4, wherein the component further comprises a needle separably coupled to the housing from the housing to assist insertion of a tip end of the sensor member into the transcutaneous position of the human body, and
wherein the terminal end portion further comprises a needle gripper configured to transfer the needle to the intermediate assembly disposed in the sensor device assembling part while moving in a state of gripping the needle.

11. A manufacturing method for a sensor device, comprising:
transferring a component from a component supplying part, in which components of a sensor device are disposed, to a sensor device assembling part in which the sensor device is assembled so as to assemble the sensor device,
wherein at least one of the components is transferred to the sensor device assembling part by a vacuum suction method, and the sensor device is assembled.

12. The manufacturing method of claim 11, wherein the component comprises:
an intermediate assembly including a housing forming an external shape of the sensor device; and
a plate-shaped sensor member disposed on the housing such that a tip end thereof is inserted into a transcutaneous position of a human body to detect biological information, and
wherein the manufacturing method comprises:
transferring the sensor member to the intermediate assembly disposed in the sensor device assembling part by the vacuum suction method to assemble the sensor device.
